# EUROPEAN PATENT SPECIFICATION

(11) **EP 2 215 107 B1**
(45) Date of publication and mention of the grant of the patent: **17.10.2012**
(21) Application number: 08849427.3
(22) Date of filing: 05.11.2008
(51) Int. Cl.: A61K 9/127, A61K 47/48, A61K 49/00, C07K 7/06, A61K 38/04, B82Y 5/00

(54) **PEPTIDE LIGAND DIRECTED DRUG DELIVERY**
AUF PEPTIDLIGAND GERICHTETE WIRKSTOFFFREISETZUNG
DÉLIVRANCE DE MÉDICAMENTS DIRIGÉE PAR UN LIGAND PEPTIDIQUE

(30) Priority: 05.11.2007 WO PCT/CN2007/003129
(43) Date of publication of application: 11.08.2010
(73) Proprietor: Shanghai Jiaotong University, Shanghai 200240 (CN)
(72) Inventor: XU, Yuhong, Minhang District, Shanghai 200240 (CN); SONG, Shuxian, Minhang District, Shanghai 200240 (CN); LIU, Dan, Minhang District, Shanghai 200240 (CN)
(74) Representative: Sweetinburgh, Mark Roger
(86) International application number: PCT/CN2008/001847
(87) International publication number: WO 2009/062399

(56) References cited:
- CN-A- 1 582 900
- CN-A- 1 617 740
- CN-A- 1 676 166
- US-A- 5 726 023
- US-A1- 2008 146 511
- A K GALANDE ET AL.: "Potent interactions of LXXLL-based protein-protein interactions" CHEMBIOCHEM - A EUROPEAN JOURNAL OF CHEMICAL BIOLOGY., vol. 6, no. 12, December 2005 (2005-12), pages 1991-1998, XP002599559 WILEY VCH, WEINHEIM. ISSN: 1439-4227
- Z LI ET AL.: "Identification and characterizaion of a novel peptide ligand of epidermal growth factor receptor for targeted delivery of peptides" FASEB JOURNAL, vol. 19, no. 12, December 2005 (2005-12), pages 1978-1985, XP002599560 FED. OF AMERICAN SOC. FOR EXPERIMENTAL BIOLOGY ISSN: 0892-6638
- '2006 International Conference on Biomedical and Pharmaceutical Engineering. 2006', vol. 1-2, ISBN 978-981-05... article SONG, S. X. ET AL.: 'Peptide ligand targeted delivery to EGFR expressing cancer cells in vitro and in vivo.', pages 490 - 493
- CHIU, G. N. C. ET AL.: 'Encapsulation of doxorubicin into thermosensitive liposomes via complexation with the transition metal manganese.' JOURNAL OF CONTROLLED RELEASE. vol. 104, no. 2, 18 May 2005, ISSN 0168-3659 pages 271 - 288
- DENG, H. W. ET AL.: 'A novel small molecule ligand for epidermal growth factor receptor targeting.' PROG. BIOCHEM. BIOPHYS. vol. 32, no. 2, 2005, ISSN 1000-3282 pages 180 - 186
- LI, Z. H. ET AL.: 'Identification and characterization of a novel peptide ligand of epidermal growth factor receptor for targeted delivery of therapeutics.' THE FASEB JOURNAL. vol. 19, no. 14, December 2005, ISSN 0892-6638 pages 1978 - 1985

## Description

### BACKGROUND OF THE INVENTION

In cancer therapy, chemotherapeutics are widely used but their efficacies are often undermined by serious side effects resulting from drug toxicities to normal tissues. In order to improve the therapeutic indexes of chemotherapeutic agents, many studies have been focusing on strategies to specifically deliver drugs to the tumor tissue while avoiding normal tissue.

Several liposome based anticancer drug formulations have been developed and are already being used in patients with better efficacies and less side effects than the nonliposomal formulations. One of the most successful products, Doxil (also known as Caelyx) (1), contains PEG coated liposomes (stealth liposomes) with extended serum half-life and the ability to gradually extravasate through the leaky vasculatures to accumulate in tumors. In addition to such a passive targeting mechanism, active targeting strategies were also proposed, in which antibodies or targeting ligands were used to direct the liposomes to further encourage the interaction between drug loaded liposomes and tumors. Antibody targeted immunoliposomes have been studied vigorously and look promising in both animal and clinical studies (2-6). Meanwhile, small molecule ligands (7, 8) and peptides (9, 10) are also being examined to construct active targeting liposomes for cancer treatment.

Compared to *in silico* or *in vitro* binding studies, the *in vivo* environment for binding is much more complex with many anatomical barriers and interference from natural clearance mechanisms such as the reticuloendothelial system (RES) and other nonspecific interactions. Many variables have been identified using immunoliposome and small molecule targeted liposomes (33, 34). Several factors have been identified that influence the pharmacokinetic properties and extravasation behavior of various active targeted liposomes (33, 34). Immune clearance against the surface conjugated ligands was a major concern (35,36). IgG containing immunoliposomes were found to interact with Fc receptors and cleared quickly (37, 38, 39). Even small molecule ligand folate conjugation caused enhanced RES clearance of liposomes in vivo (8). In addition, both surface ligand densities (40) and the length of PEG linkers (8, 19) were also found to be important.

The epidermal growth factor receptor (EGFR) is over-expressed in a wide variety of human cancers, including, but not limited to: lung, breast, bladder, and ovarian cancers. It has also been found to associate with various features of advanced disease and poor prognosis (11). Various EGFR targeting vectors and conjugates have been reported to enable increased delivery of cytotoxic drugs, toxins, or radionuclides to cancer cells and inhibit tumor growth in animal models (12-16). Mamot and colleagues developed an EGFR specific immunoliposome and showed in a series of studies that it has better delivery properties and antitumor efficacies in vitro and in vivo (17-19).

There have been several successful drugs developed targeting EGFR, including the tyrosine kinase inhibitors Tarceva® and Iressa®, and its blocking antibody Erbitux®. In addition to designing drugs directed against EGFR, another strategy is to develop systems that can direct existing therapeutic agents towards it. Many studies have exploited the use of its natural ligand, epidermal growth factor (EGF), for targeted delivery of cytotoxic drugs, toxins, liposomes and other drug/gene delivery systems, and radionuclide systems (12-15). But the endogenous pro-proliferation effect of EGF on cancer cells is always a concern. Two other strategies were proposed. One is to use antibodies or antibody fragments to direct binding to EGFR (17, 18, 27), and the other is to use smaller peptides or EGF fragments (12, 28, 29). Small peptides that can strongly and specifically bind to EGFR but with minimal immunogenicity and pro-proliferation effects are quite desirable.

FASEB Journal 19(12), December 2005, pages 1978-1985 discloses peptides able to bind specifically the EGF receptor (EGFR) and which are used for the same purpose.

There remains a need in the art for materials and methods to effectively deliver therapeutic agents to target cells, for example, those overexpressing the EGFR. This need and others are met by the present invention.

### SUMMARY OF THE INVENTION

The present invention provides-peptides that can be used to deliver agents, for example, therapeutic agents and/or imaging agents to a desired target cell. In one embodiment, the present invention provides a peptide comprising a sequence selected from the group consisting of SEQ ID NO: 1, SEQ ID NO:3, SEQ ID NO: 4, SEQ ID NO:5, SEQ ID NO:6, SEQ ID NO:7, and SEQ ID NO: 8, wherein the peptide is from 6 to .. : 15 amino acids in length and wherein Xaa₆ of SEQ ID NO: 8 is selected from the group consisting of serine, asparagine and cysteine. In one embodiment, the peptide may comprise the sequence LARLLT (SEQ ID NO:1). Peptides of the invention may be six, seven, eight, nine, ten or more amino acids in length. In some embodiments, peptides of the invention are six amino acids in length and consist of a sequence selected from the group consisting of SEQ ID NO:1, SEQ ID NO:3, SEQ ID NO:4, SEQ ID NO:5, SEQ ID NO:6, SEQ ID NO:7, and SEQ ID NO:8 wherein Xaa₆ of SEQ ID NO: 8 is selected from the group consisting of serine, asparagine and cysteine. In some embodiments a peptide of the invention may consist of the amino acid sequence LARLLT. Also described are peptidomimetics that mimic the EGFR binding activity of the peptides of the invention as well as substitutions of amino acids in the peptide that conserve its function.

In another embodiment, peptides : of the invention may be attached, directly cr indirectly, to one or more compounds. In some embodiments, a peptide of the invention is attached to a therapeutic and/or an imaging agent. In one embodiment, the present invention provides a molecule comprising a lipid and a peptide of the invention.
In some embodiments the peptide may comprise the sequence LARLLT. Optionally, the peptide may be attached to the lipid indirectly through the use of a linker. Any linker known to those skilled in the art may be used. Examples of suitable lipids to which the peptides of the invention may be attached include, but are not limited to, 1,2-Distearoyl-sn-Glycero-3-Phosphoethanolamine (DSPE). In some embodiments, a linker may comprise polyethylene glycol (PEG). An example of a lipid attached to a linker is 1,2-Distearoyl-sn-Glycero-3-Phosphoethanolamine-N-[Methoxy(Polyethylene glycol)-2000] (DSPE-PEG2000). Such a molecule may be further attached to one or more peptides of the invention.

Peptides of the invention may be attached to an active agent, for example, a therapeutic agent and/or an imaging agent. The attachment may be direct or indirect. Peptides of the invention may be attached to active agents by directly reacting the peptide with a functional group on the active agent. Peptides of the invention may be indirectly attached to an active agent by reacting the peptide and the active agent with a linker. Typically, a linker will be a bifunctional molecule capable of forming a covalent attachment with both the peptide and the active agent.

In another embodiment, the present invention comprises liposomes. An example of a liposome of the invention is a liposome comprising a molecule comprising a lipid and a peptide of the invention.
In some embodiments the peptide may comprise the sequence LARLLT. As discussed above, molecules for use in the liposomes of the invention may comprise a linker between a lipid moiety and a peptide moiety. In one embodiment, the lipid may be of 1,2-Distearayl-sn-Glycero-3-Phosphoethanolamine (DSPE). In one embodiment, the linker may be polyethylene glycol (PEG). In one embodiment, a lipid-linker-peptide combination may be 1,2-Distearoyl-sn-Glycero-3-Phosphoethanolamine-N-[Methoxy(Polyethylene glycol)-2000] (DSPE-PEG2000)-LARLLT. Liposomes of the invention may be prepared using any lipid or combination of lipids known to those skilled in the art. Examples of suitable lipids that may be used to prepare liposomes include, but are not limited to, phospholipids (e.g., phosphatidyl cholines, phosphatidyl glycerols, and phosphatidyl ethanolamines), lysolipids and PEGylated phospholipids. Suitable lipids may be combined in any ratio. Optionally, a liposome for use in the invention may be a thermosensitive liposome comprised of lipids that have a gel to liquid phase transition temperature of from about 39.0°C to about 45°C. One example of a suitable liposome may comprise DPPC:MSPC at a ratio of 99:1, 98:2, 97:3, 96:4, 95:5, 90:10, to about 80:20, 75:25, 70:30, 65:35, 60:40, or even 51:49 on a mole percentage basis. One or more additional lipids (e.g., the peptide-linker-lipid and/or PEGylated lipids) may be incorporated into thermosensitive liposomes.

Liposomes of the invention may further comprise one or more compounds selected from the group consisting of therapeutic compounds and imaging compounds. Typically, a liposome of the invention comprises a bilayer and defines an interior space and the compound is in the interior space of the liposome, in the bilayer of the liposome, or is in both the interior space and bilayer. In some embodiments, a compound may be associated with the exterior of the liposome. One example of a suitable type of compound for inclusion in the liposomes of the invention is an anticancer compound. Examples of suitable anticancer compounds include, but are not limited to, alkylating agents, antimetabolites , spindle poison plant alkaloids, cytotoxic antitumor antibiotics, topoisomerase inhibitors, monoclonal antibodies or fragments thereof, photosensitizers, kinase inhibitors, antitumor enzymes and inhibitors of enzymes, apoptosis-inducers, biological response modifiers, anti-hormones, retinoids and platinum containing compounds.. In some embodiment, a liposome of the invention may comprise docetaxel. In some embodiments, a liposome of the invention may comprise doxorubicin. In some embodiments, a liposome of the invention may comprise a platinum containing compound, for example, carboplatin or cisplatin.

The present invention also provides a compound for imaging associated with a peptide of the invention.
In some embodiments the peptide may comprise the sequence LARLLT. Association may be covalent or non-covalent. In some embodiments, the peptide may be directly or indirectly attached to an active agent. In some embodiments, the peptide is attached to a lipid and the peptide-attached lipid is in the form of a liposome. The compound to be delivered may be part of the liposome, for example, in the lipid bilayer or may be contained in the interior space of the liposome. Alternatively, the peptide may be attached to the compound and the compound may be included in the liposome, for example, in the lipid bilayer or in the interior space. When the peptide is attached to a lipid, the peptide-attached lipid may comprise a linker between the lipid and the peptide. An example of a suitable lipid for use in the methods of the invention is 1,2-Distearoyl-sn-Glycero-3-Phosphoethanolamine (DSPE). A suitable linker may comprise polyethylene glycol (PEG). Thus, one example of a lipid attached to a peptide of the invention through a linker is 1,2-Distearoyl-sn-Glycero-3-Phosphoethanolamine-N-[Methoxy(Polyethylene glycol)-2000] (DSPE-PBG2000)-LARLLT, which may be incorporated into liposomes and used in the methods of the invention. Any other lipids known to those skilled in the art may be included in the liposomes comprising a lipid-linker-peptide of the invention. In some methods of the invention, a compound may be associated with a liposome comprising a peptide-linker-lipid as described above and the liposome may have a gel to liquid phase transition temperature of from about 39.0°C to about 45°C. Such a liposome may be administered to a subject in need thereof and a portion of the subject may be heated to a temperature above the gel to liquid phase transition temperature of then liposome.

Any suitable means of healing the target tissue may be used, for example, application of radiofrequency radiation, application of ultrasound which may be high intensity focused ultrasound, application of microwave radiation, sources that generate infrared radiation such as a warm water bath, light, as well as other forms of externally and internally applied radiation such as that generated by radioisotopes, or electrical and magnetic fields.

Any compound may be administered using the methods described herein. Suitable examples of compounds that may be administered include therapeutic compounds and imaging compounds, which may be directly or indirectly attached to a peptide of the invention or may be included in a liposome comprising a lipid linked to a peptide of the invention. In one method; the liposome may comprise a bilayer and define an interior space and the compound may be in the interior space of the liposome, in the bilayer of the liposome, or in both the interior space and bilayer. Also described herein are methods to deliver therapeutic compounds, for example, anticancer compounds. Anticancer compounds include, but are not limited to, alkylating agents, antimetabolites, spindle poison plant alkaloids, cytotoxic antitumor antibiotics, topoisomerase inhibitors, monoclonal antibodies or fragments thereof, photosensitizers, kinase inhibitors, antitumor enzymes and inhibitors of enzymes, apoptosis-inducers, biological response modifiers, anti-hormones, retinoids and platinum containing compounds. In some examples, the therapeutic methods may be used to deliver docetaxel. In some examples, the therapeutic methods may be used to deliver doxorubicin. In some examples, the therapeutic methods may be used to deliver a platinum containing compound, for example, carboplatin or cisplatin.

In further embodiments, the present invention provides liposomes and peptides for use in treating cancer. Such treatment may comprise administering to a subject in need thereof an anticancer compound in association with a peptide of the invention. One example of association is to attach a peptide of the invention directly or indirectly to the anticancer compound. Another example is to include the anticancer compound in a liposome wherein the liposome comprises a lipid associated with a peptide of the invention.
In some embodiments the peptide may comprise the sequence LARLLT. Typically, the peptide is attached to the lipid directly or through a linker. A suitable lipid is 1,2-Distearoyl-sn-Glycero-3-Phosphoethanolamine (DSPE). A suitable linker is polyethylene glycol (PEG). Thus, one example of a peptide-linker-lipid that may be incorporated into a liposome and the liposome used to deliver an anticancer compound is 1,2-Distearoyl-sn-Glycero-3-Phosphoethanolomine-N-[Methoxy(Polyethylene glycol)-2000] (DSPE-PEG2000)-LARLLT. Examples of suitable lipids that may be used to prepare liposomes include, but are not limited to, phospholipids (e.g., phosphatidyl cholines, phosphatidyl glycerols, and phosphatidyl ethanolamines), lysolipids and PBGylated phospholipids. Optionally, a liposome for use in methods of heating cancer of the invention may have a gel to liquid phase transition temperature of from about 39.0°C to about 45°C. One example of a suitable liposome may comprise DPPC:MSPC at a ratio of 99: 1, 98:2, 97:3, 96:4, 95:5, 90:10, to about 90:20, 75:25, 70-30, 65:35, 60:40, or even 51:49 on a mole percentage basis. Into such liposomes the peptide-linker-lipid may be incorporated. Any known anticancer agent may be used including, but not limited to, alkylating agents, antimetabolites, spindle poison plant alkaloids, cytotoxic antitumor antibiotics, topoisomerase inhibitors, monoclonal antibodies or fragments thereof, photosensitizers, kinase inhibitors, antitumor enzymes and inhibitors of enzymes, apoptosis-inducers, biological response modifiers, anti-hormones, retinoids and platinum containing compounds. In some embodiments, the anticancer compound may be docetaxel. In some embodiments, the anticancer compound may be doxorubicin. In some embodiments, the anticancer compound may be a platinum containing compound, for example, carboplatin. In some methods of treating cancer an anticancer compound may be associated with a liposome comprising a peptide-linker-lipid as described above and the liposome may have a gel to liquid phase transition temperature of from about 39.0°C to about 45°C. Such methods may further comprise heating a portion of the subject. Any method of heating the subject may be used, for example, application of microwave energy, other electromagnetic wave energy, radio frequency ablation, high intensity focused ultrasound, ultrasound, application of heated water and the like.

### BRIEF DESCRIPTION OF THE DRAWINGS

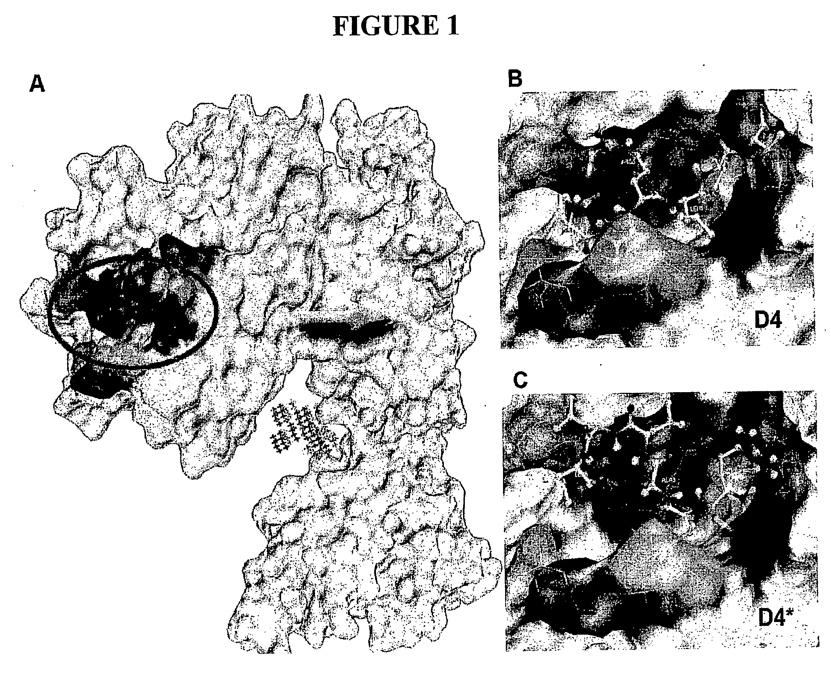

Figure 1. Docked structures of LARLLT and control peptide on EGFR. A. EGFR structure model from PDB. The asterisk-filled area is the EGF binding site. The area circled is the binding pocket we selected for docking. B. The lowest energy docked conformation of LARLLT inside the EGFR pocket. The peptide is shown in the ball-stick mode. Potential hydrogen bonds are indicated as cylinders. C. the lowest energy docked conformation of control peptide inside the EGFR pocket.

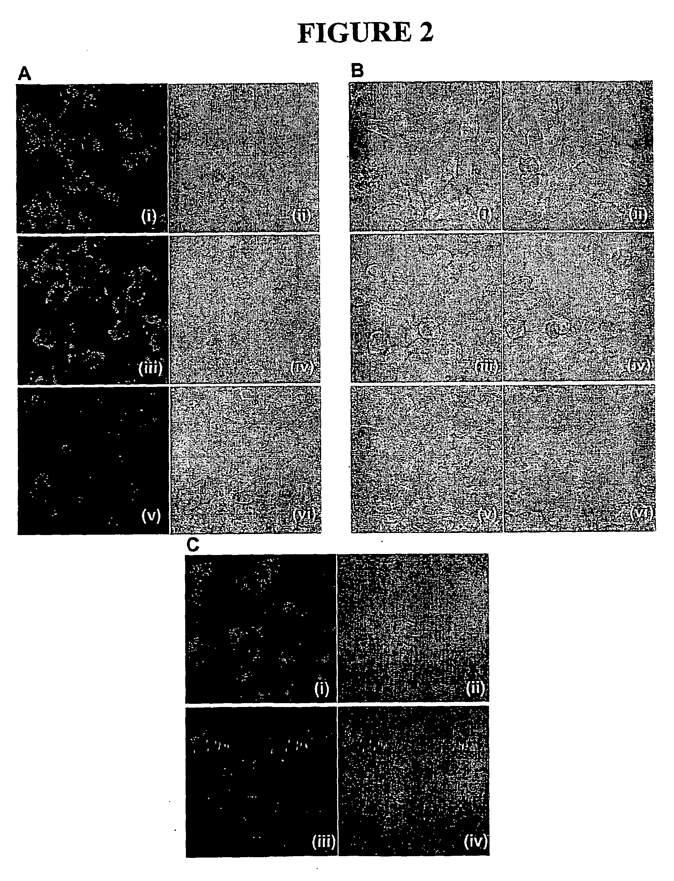

Figure 2. Fluorescence microscopy studies of ligand directed liposome binding to EGFR high expressing cells. Panel A: Binding of LARLLT, control peptide and EGF targeted liposomes to H1299 cells. (i) LARLLT liposomes, (iii) EGF liposomes, (v) control peptide liposomes. (ii), (iv), (vi) are the phase contrast micrograph of the same fields in (i), (ii), (v). Panel B: Binding of LARLLT targeted liposomes to H1299 at 4°C or 37°C in the presence of 50x mole excess free ligands. (i) binding at 37°C with excess free LARLLT, (ii) binding at 37°C without free LARLLT, (iii) binding at 4°C with excess free LARLLT. (iv) binding at 4°C without free LARLLT, (v) binding at 37°C with excess free EGF, and (vi) binding at 37°C without free EGF. Panel C: Binding of LARLLT, control peptide liposomes to SPC-A1 cells. (i) LARLLT liposomes, (iii) control peptide liposomes. (ii) and (iv) are the phase contrast micrograph of the same fields in (i) and (iii).

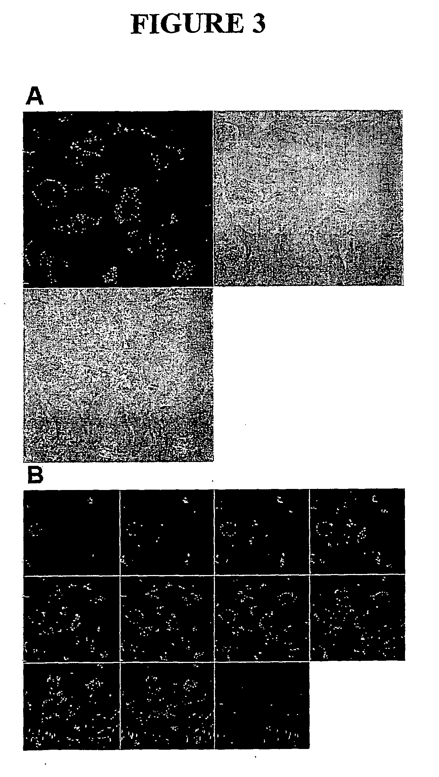

Figure 3. Internalization of LARLLT-conjugated liposome by H1299 cells. A. Overlay of phase contrast and fluoresence images of endocytosed LARLLT liposomes. B. 11 slices from the top to the bottom of the cells using the Z stack scan mode of confocal fluorescence microscope.

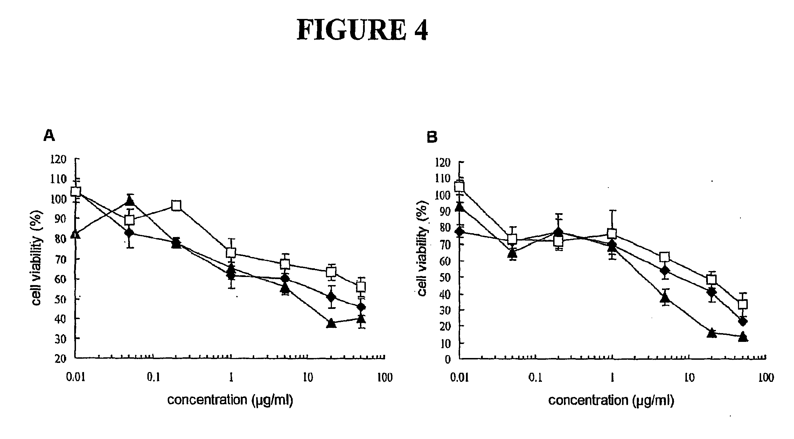

Figure 4. Cell killing effects of targeted liposomes containg doxorubicin. A. H1299 cells. The calculated IC50 for with LARLLT liposomes (filled diamond) was ~13 µg/ml, for control peptide lipsomes (open square) was 85 µg/ml], and for free doxorubicin alone (filled triangle) was 5.7 µg/ml. B. SPCA1 cells. The calculated IC50 for with LARLLT liposomes (filled diamond) was~5µg/ml, for control peptide lipsomes (open square) was 15µg/ml, and for free doxorubicin alone (filled triangle) was 2µg/ml.

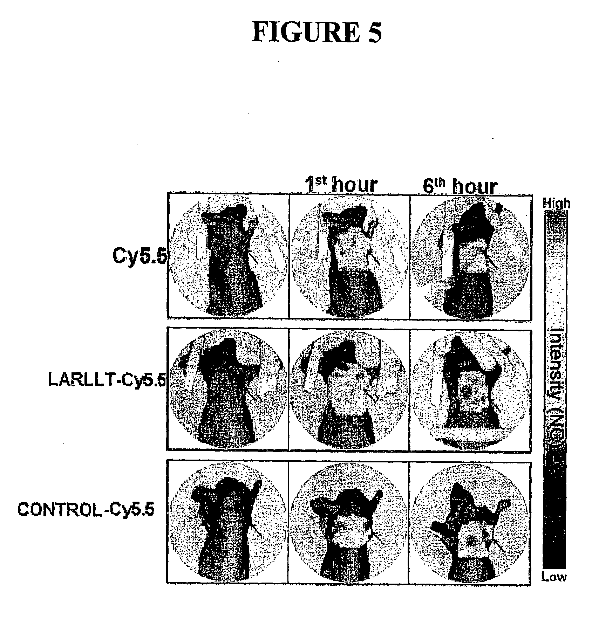

Figure 5. The fluorescence image of Cy5.5 and Cy5.5 labeled peptides in tumor bearing mice. Images shown were taken at 1 hour and 6 hours after injection of free Cy5.5 dye, LARLLT-Cy5.5 and control peptide-Cy5.5.

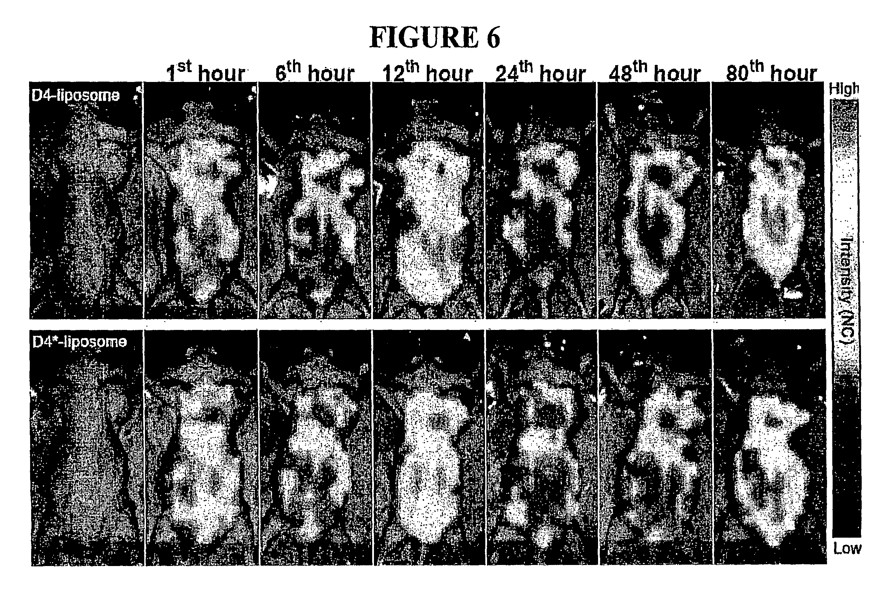

Figure 6. The fluorescence images of peptide directed liposome distribution and accumulation in tumor tissues. Images shown (from left to right) were the light picture of the mouse, fluorescence images taken at 1 hour, 6 hours, 12 hours, 24 hours, 48 hours, 80 hours after the injection of LARLLT liposomes (top row) and control peptide liposomes (bottom row).

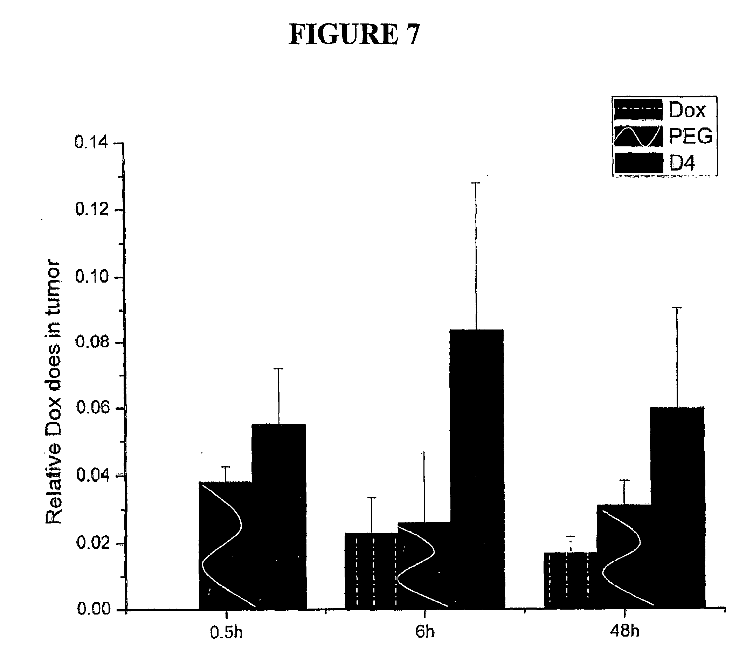

Figure 7 is a bar graph showing the accumulated doxorubicin concentration in xenograft H460 tumor mass in nude mice at various time points after drug injection. The solid black bars represented the tumor doxorubicin concentration after injection of doxorubicin solution. The black bars with dashed lines represented the tumor doxorubicin concentration after injection of regular doxorubicin liposomes, the black bars with wavy lines represented the tumor doxorubicin concentration after injection of LARLLT modified doxorubicin liposomes.

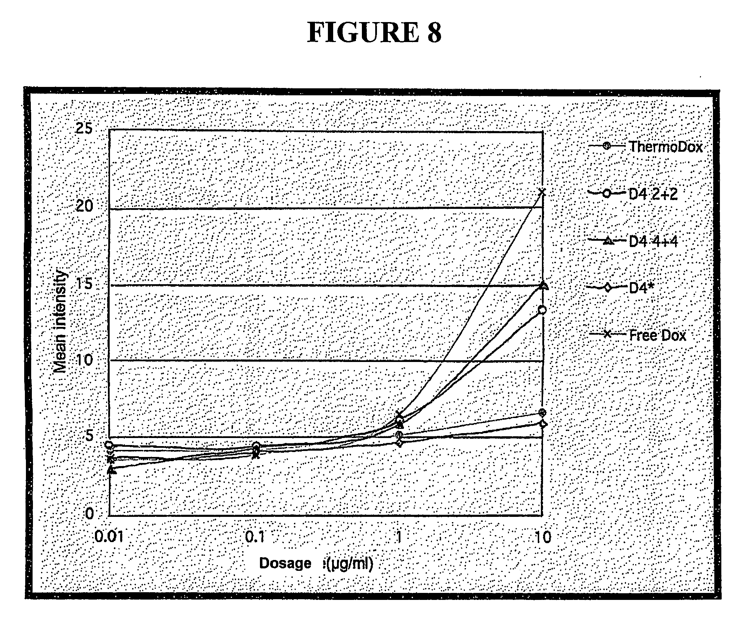

Figure 8 is line graph showing observed fluorescence of H460 cells treated with various doxorubicin-containing preparations at 0.01 to 10 µg/ml doxorubicin. x = free doxorubicin, 0 = D4* liposomes, ▲ =D4 4+4 liposomes, o = D4 2+2 liposomes, **•** = Thermodox liposomes. D4 is is a liposome comprising SEQ ID NO:1 and D4* is a liposome comprising SEQ ID NO:2. D4-ThermoDox2+2, is comprised DPPC:MSPC: DSPE-MPEG2000:D4-DSPE-MPEG2000 in a 90:10:2:2 molar ratio and D4-ThermoDox4+4, is comprised DPPC:MSPC: DSPE-MPEG2000:D4-DSPE-MPEG2000 in a 90:10:4:4 molar ratio. D4*-ThermoDox comprised DPPC:MSPC: DSPE-MPEG2000:D4*-DSPE-MPE02000 in a 90:10:2:2 molar ratio.

### DETAILED DESCRIPTION OF THE INVENTION

Peptides

The present invention provides small peptides that can strongly and specifically bind to EGFR. Typically, peptide. - of the invention can bind to the EGFR without inducing immunogenic and/or pro-proliferative effects.

An exemplary peptide of the invention is a peptide that comprises the amino acid sequence Leu Ala Arg Leu Leu Thr (SEQ ID NO: 1). Additional examples of peptides of the invention include, but are not limited to, peptides wherein one or more amino acids of SEQ ID NO:1 have been substituted with a different amino acid. In some embodiments, only one position of SEQ ID NO:1 will be substituted. In some embodiments, more than one position of SEQ ID NO:1 will be substituted. Substitutions may be made at any position of SEQ ID NO: 1. In some embodiments, one or more positions of SEQ ID NO: 1 will be substituted with one or more naturally occurring amino acids. In some embodiments, one or more positions SEQ ID NO:1 will be substituted with one or more non-naturally occurring amino acids. In some embodiments, a peptide of the invention may comprise one or more D-amino acids.

Typically, substitutions of amino acids in the peptides of the invention may be conservative substitutions. As used herein, a conservative substitution is the replacement of one amino acid with another amino acid of similar physical and/or chemical characteristics. The standard genetically coded amino acids can be grouped according to their characteristics, particularly of polarity and charge. One convenient grouping is as follows: glycine and alanine; serine, threonine, asparagine, glutamine and cysteine; lysine, arginine and histidine; aspartic acid and glutamic acid; valine, leucine, isoleucine, and methionine; and phenylalanine, tryptophan and tyrosine. Replacement of an amino acid from one group with another amino acid in the same group is referred to as a "conservative substitution". Such substitutions do not generally materially affect the properties of the peptides of the invention, for example, the ability to bind to EGFR. Peptides of the invention include peptides that differ from SEQ ID NO:1 only by one or more conservative substitutions.

Examples of peptides of the invention include, but are not limited to:

Leu Ala Arg Leu Leu Thr (SEQ ID NO:1)

Xaa₁ Ala Arg Leu Leu Thr (SEQ ID NO:3)

Leu Xaa₂ Arg Leu Leu Thr (SEQ ID NO:4)

Leu Ala Xaa₃ Leu Leu Thr (SEQ ID NO:5)

Leu Ala Arg Xaa₄ Leu Thr (SEQ ID NO:6)

Leu Ala Arg Leu Xaa₅ Thr (SEQ ID NO:7)

Leu Ala Arg Leu Leu Xaa₆ (SEQ ID NO: 8)

were Xaa₁ is selected from the group consisting of valine, isoleucine, and methionine; Xaa₂ is glycine; Xaa₃ is selected from the group consisting of lysine and histidine; Xaa₄ is selected from the group consisting of valine, isoleucine, and methionine; Xaa₅ is selected from the group consisting of valine, isoleucine, and methionine; and Xaa₆ is selected from the group consisting of serine, asparagine, and cysteine. The present invention also contemplates peptides comprising more than one of the above substitutions.

Also described herein are non-peptide compounds showing the same ability to bind to EGFR as the peptides of the invention. Such peptidomimetics or "small molecules" capable of mimicking the activity of the peptides of the invention may be used in the methods described herein. Those skilled in the art are able to design peptidomimetics using well knows techniques such as those described in Fauchere J., Adv. Drug Res. 15: 29 (1986); and Evans et al., J. Med. Chem. 30: 1229 (1987).

Peptidomimetics that have the ability to bind the EGFR may be used analogously as the peptides of the invention. Typically, such peptidomimetics have one or more peptide linkages optionally replaced by a linkage which may convert desirable properties such as resistance to chemical breakdown in vivo. Such linkages include, but are not limited to, --CH₂NH--, --CH₂S--, --CH₂CH₂ --, --CH=CH--, COCH₂ --, --CH(OH)CH₂--, and -CH₂SO--.

Peptides of the invention may be associated with any desired material to deliver the material to a desired target cell. Examples of suitable materials include, but are not limited to, compounds (e.g., active agents, therapeutic agents, imaging agents etc.), liposomes (e.g., thermosensitive liposomes and stealth liposomes) and nanoparticles (e.g., protein nanoparticles, metal nanoparticles, and nanoparticles of crystallized small molecules).

Association of the peptides of the invention with material to be delivered may be covalent or non-covalent and direct or indirect. In some embodiments, a peptide of the invention may be covalently attached to a material to be delivered, for example, through a linker.

Examples of linkers are cross linking agents capable of covalently attaching to a peptide of the invention and to a material to be delivered. Typically crosslinkers react with functional moieties (e.g., -OH, NH₂, COOH and, -SH groups) present on the peptide of the invention and the material to be linked. Different crosslinkers may be chosen because of their different ability to react with different functional moieties.

the techniques of polypeptide conjugation or coupling through activated functional groups presently known in the art are particularly applicable. See, for example, Aurameas, et al., Scand J. lmmunol., Vol. 8, Suppl. 7:7-23 (1978) and U.S. Pat. Nos. 4,493,795, 3,791,932 and 3,839,153
for the disclosure of coupling of peptides. In addition, a site-directed coupling reaction can be carried out so that any loss of activity due to polypeptide orientation after coupling can be minimized. See, for example, Rodwell et al., Biotech., 3:889-894 (1985), and U.S. Pat. No .4,671,958. Exemplary additional linking procedures include the use of Michael addition reaction products, di-aldehydes such as glutaraldehyde, Klipstein, et al., J. Infect. Dis., 147:318-326 (1983) and the like, or the use of carbodiimide technology as in the use of a water-soluble carbodiimide to form amide links. Alternatively, the heterobifunctional cross-linker SPDP (N-succinimidyl-3-(2-pyridyldithio) proprionate)) can be used to conjugate peptides, in which an N-or C-terminal cysteine has been introduced.

The conjugation of the peptide to an active agent as set forth herein, can be effected by chemical conjugation procedures well known in the are, such as by creating peptide linkages, use of condensation agents, and by employing well known bifunctional cross-linking reagents. The conjugation may be direct, which includes linkages not involving any intervening group, e.g., direct peptide linkages, or indirect, wherein the linkage contains an intervening moiety, such as a protein or peptide, e.g., plasma albumin, or other spacer molecule. For example, the linkage may be via a heterobifunctional or homobifunctional cross-linker, e.g., carbodiimide, glutaraldehyde, N-succinimidyl 3-(2-pyridydithio) propionate (SPDP) and derivative, bis-maleimide, 4-(N-maleimidomethyl) cyclohexane-1-carboxylate, and the like. Cross-linking may also be accomplished without exogenous cross-linkers by utilizing reactive groups on the molecules being conjugated. Methods for chemically cross-linking peptide molecules are generally known in the art, and a number of hetero- and homobifunctional agents are described in, e.g., U.S.Pat. Nos. 4,3 55,023, 4,657,853, 4,676,980,4,925,921, and 4,970,156, and additional cross linking reagents are commercially available, for example, from Pierce Chemical Co., Rockford, IL. 61105, Cleavable cross-linkers, particularly those that form cleavable disulfide bonds, may be employed to allow cleavage of the conjugate to free the therapeutic agent under physiological conditions. An example of such a cleavable cross-linker is 4-succinimidyloxycarbonyl-a-(2-pyridyldithio)-toluene. Such conjugation, including cross-linking, should be performed so as not to substantially affect the desired function of the peptide or active agent conjugated thereto.

In one example, peptides and peptidomimetics may be attached to nanoparticles. Many varieties of nanoparticles are available, such as different polymeric and metal nanoparticles, liposomes, niosomes, solid lipid particles, micelles, quantum dots, dendrimers, microcapsules, cells, cell ghosts, lipoproteins, and different nanoassemblies. Protein nanoparticles may be prepared using any technique known in the art, for example, using a modified desolvation-crosslinking method. A protein in aqueous solution (2%, w/v) can be incubated with an active agent in buffer and then added dropwise into ethanol to form protein nanoparticles incorporating an active agent. These may be crosslinked to the peptide of the invention, for example, using glutaraldehyde. Gold nanoparticles can be synthesized according to standard wet chemical methods using sodium borohydride as a reducing agent.

In one embodiment, peptides of the invention may be attached to a lipid including, but not limited to, attached directly to the headgroup region of the lipid, or attached via a linker, such as a polymer (e.g., PEG), to the headgroup region, and incorporated into a liposome. The liposome (which may be a thermosensitive liposome, i.e., a liposome with a gel to liquid phase transition temperature of from about 39.0°C to about 45°C) will typically comprise an active agent (e.g., therapeutic agent and/or imaging agent.)

Liposomes of the invention typically comprise one or more phosphatidylcholines. Suitable examples of phosphatidylcholines that can be used in the practice of the invention include, but are not limited to, 1,2-Dilauroyl-sn-glycero-3-phosphocholine (DLPC), 1,2-Dimyristoyl-sn-glycero-3-phosphacholine (DMPC), 1,2-Dipalmitoyl-sn-glycero-3-phosphocholine (DPPC), 1,2-Distearoyl-sn-glycero-3-phosphocholine (DSPC), 1,2-Dioleoyl-sn-glycero-3-phosphocholine (DOPC), and 1-Palmitoyl-2-oleoyl-sn-glycero-3-phosphocholine (POPC).

Liposomes of the invention typically comprise one or more phosphatidylglycerols. Suitable examples of phosphatidylglycerols include, but are not limited to, 1,2-Dimyristoyl-sn-glycero-3-phosphoglycerol (DMPG), 1,2-Dipalmitoyl-sn-glycero-3-phosphoglycerol (DPPG), 1,2-Distearoyl-sn-glycero-3-phosphoglycerol (DSPG), and 1-Palmitoyl-2-oleoyl-sn-glycero-3-phosphoglycerol (POPG).

Liposomes of the invention typically comprise one or more lysolipids. As used herein "lysolipid" refers to any derivative of phosphatidic acid (1,2-diacyl-sn glycero-3-phosphate) that contains only one acyl chain covalently linked to the glycerol moiety. Derivatives of phosphatidic acid include, but are not limited to, phosphatidylcholine, phosphatidylglycerol, and phosphatidylethanolamine. Any lysolipid known to those skilled in the art may be used in the practice of the invention. Lysolipids include, but are not limited to, monopalmitoylphosphatidylcholine (MPPC), monolaurylphosphatidylcholine (MLPC), monomyristoylphosphatidylcholine (MMPC), monostearoylphosphatidylcholine (MSPC), and mixtures thereof.

Liposomes of the invention may also comprise a lipid to which a hydrophilic polymer has been attached, for example, a lipid covalently attached to PEG.

Active agents

Peptides of the invention may be used to deliver active agents. As used herein, "active agent" includes any compound desired to be delivered to a specific site in a subject. Any active agent may be used in the practice of the invention.

Anticancer agents may be used as the active agents in the thermosensitive liposomes of the invention. Suitable examples of anticancer agents include:

alkylating agents, for example, nitrogen mustards (e.g., Chlorambucil, Chlormethine, Cyclophosphamide, Ifosfamide, Melphalan, nitrosoureas (e.g., Carmustine, Fotemustine, Lomustine, Streptozocin), platinum containing compounds (e.g., Carboplatin, Cisplatin, Oxaliplatin, BBR3464), Busulfan, Dacarbazine, Mechlorethamine, Procarbazine, Temozolomide, ThioTEPA, and Uramustine;

antimetabolites that target, for example, folic acid (e.g., aminopterin, methotrexate, pemetrexed, raltitrexed), purine metabolism (e.g., cladribine, clofarabine, fludarabine, mercaptopurine, pentostatin, thioguanine), pyrimidine metabolism (e.g., capecitabine, cytarabine, fluorouracil, floxuridine, gemcitabine);

spindle poison plant alkaloids, for example, taxanes (e.g., docetaxel, paclitaxel) and vinca (e.g., vinblastine, vincristine, vindesine, vinorelbine);

cytotoxic/antitumor antibiotics, for example, anthracycline antibiotics (e.g., daunorubicin, doxorubicin, epirubicin, idarubicin, mitoxantrone, valrubicin, carinomycin, nacetyladriamycin, rubidazone, 5-imidodaunomycin, N30 acetyldaunomycin, and epirubicin), bleomycin, mitomycin, and actinomycin;

topoisomerase inhibitors, for example, camptothecines (e.g., camptothecin, topotecan, irinotecan), podophyllum (e.g., etoposide, teniposide).

monoclonal antibodies or fragments thereof, for example, Alemtuzumab, Bevacizumab, Cetuximab, Gemtuzumab, Panitumumab, Rituximab, Tositumomab, and Trastuzumab;

photosensitizers, for example, aminolevulinic acid, methyl aminolevulinate, porfimer sodium, and verteporfin;

kinase inhibitors, for example, Dasatinib, Erlotinib, Gefitinib, Imatinib, Lapatinib, Nilotinib, Sorafenib, Sunitinib, and Vandetanib.

Enzymes, for example, asparaginase, pegaspargase and inhibitors of enzymes, for example hydroxyurea;

Apoptosis-inducers, for example, arsenic trioxide, Velcade and Genasense;

Biological response modifiers, for example, Denileukin Diftitox;

Anti-hormones, for example, Goserelin acetate, leuprolide acetate, triptorelin pamoate, Megestrol acetate, Tamoxiifen, toremifene, Fulvestrant, testolactone, anastrozole, exemestane and letrozole;

Retinoids, for example, 9-cis-retinoic acid and all-*trans*-retinoic acid

In additional embodiments, the thermosensitive liposomes of the invention can comprise more than one antineoplastic agent, or more than one thermosensitive liposome can be used in the methods of the invention, each of which comprises different active agents, for example, different anticancer agents.

Additional active agents that can be used in the practice of the present invention include, but are not limited to antibiotics, antifungals, anti-inflammatory agents, immunosuppressive agents, anti-infective agents, antivirals, antihelminthic, and antiparasitic compounds.

An active agent may be an imaging agent. Imaging agents suitable for use in the present liposome preparations include ultrasound contrast agents, X-Ray contrast agents (for example: gold and barium-based agents, Iohexol, Visipaque and other iodine-based agents, as well as other particulate agents), magnetic resonance contrast agents (for example, paramagnetic materials containing: manganese, iron, gadolinium and other lanthanides), nuclear medicine agents (such as radioisotopes or compounds containing radioisotopes).

Methods of use

Active agents associated with peptide of the invention can be administered to a subject using any suitable route, for example, intravenous intraarterial, intramuscular intraperitoneal, subcutaneous, intradermal, intraarticular, intrathecal intracerebroventricular as well as other routes such as by inhalation, orally or directly on to mucous membranes (sublingually) Any tissue can be treated using the materials of the invention. Examples of tissues which can be treating using the materials of the present invention include, but are not limited to, liver, kidney, bone, soft tissue, head and neck tissue, muscle, adrenal tissue, lung, breast, thryroid, pancreas, uterine tissues including endometrial and cervical tissue, ovary, and prostate. Tissues that can be treated include both cancerous tissue, otherwise diseased or compromised tissue, as well as healthy tissue if so desired.

The dose of active agent administered to the subject using the methods described herein: can be determined by those of skill in the art, and suitably is administered intravenously over an extended time period, for example, from about 1 minutes to about 24 hours.

The dose of active agent may be adjusted as is known in the art depending upon the active agent comprised in the carrier.

When an active agent is incorporated into a thermosensitive liposome comprising a peptide of the invention, the target tissue of the subject may be heated before and/or during and/or after administration of the thermosensitive liposomes. In one example :, the target tissue is heated first (for example, for 10 to 30 minutes) and the thermosensitive liposomes are delivered into the subject as soon after heating as practicable. In another example thermosensitive liposomes are delivered to the subject and the target tissue is heated as soon as practicable after the administration This heating may continue for up to 3 hours.

Any suitable means of heating the target tissue may be used, for example, application of radiofrequency radiation, application of ultrasound which may be high intensity focused ultrasound, application of microwave radiation, any source that generates infrared radiation such as a warm water bath, light,, as well as other forms of externally and internally applied radiation such as that generated by radioisotopes, orelectrical and magnetic fields.

Having now described the present invention in detail, the same will be more clearly understood by reference to the following examples, which are included herewith for purposes of illustration only and are not intended to be limiting of the invention.

### EXAMPLES

### EXAMPLE 1

Egg Phosphatidylcholine, 1,2-Distearoyl-sn-Glycero-3-Phosphoethanolamine (DSPE), 1,2-Distearoyl-sn-Glycero-3-Phosphoethanolamine,N-[Metboxy(Polyethylene glycol)-2000] (DSPE-PEG2000} 1,2-Distearoyl-sn-Glycero-3-Phosphoethanolamine-N-[Maleimide(Polyethylene Glycol)2000] (DSPE-PEG2000-Mal) and Cholesterol were all purchased from Avanti Polar Lipids (AL, USA). Lissamine™ rhodamine B 1,2-dihexadecanoyl-sn-glycero-3-phosphoethanolamine (rhodamine DHPE) and N-(fluorescein-5-thiocarbamoyl)-1,2-dihexadecanoyl-sn-glycero-3-phosphoethanolamine (fluorescein DHPE) were purchased from Invitrogen Corporation. N-Succinimidyl 3-(2-pyridyldithio) propionate (SPDP) and tris(2-carboxyethyl) phosphine (TCEP) were purchased from Pierce Biotechnology, Inc. Cy5.5 Mono NHS Ester was supplied by GE Healthcare. All other chemicals in analytical grade were obtained from Sinopharm Chemical Reagent Co. Ltd (Shanghai, China).

The crystal structure of EGFR in inactive state was downloaded from PDB (http://www.rcsb..org/pdb/, code:1NQL). A 3D structure model was built and scanned for candidate binding pocket using the PSCAN 2.2.2 program provided by Dr. Sheng-Hung Wang (http://home.pchome.com.tw/team/gentamicin/mol/ mol.htm). A pocket on the surface near the top was selected and shown in Figure 1. The amino acid residue structure inside the pocket was carefully analyzed and a focused library containing 132 hexapeptides was designed based on the Mekler-Idlis amino acid pairing theory (20).

The library was screened and ranked for binding to the EGFR surface pocket using Autodock3.0. In order to speed up the calculation and run the program on multiple processors, we modified the software to enable parallel computing. All the computing was done on the SGI Onyx3800 machine in SJTU supercomputing center. The 132 peptides were at first roughly screened to select 50 peptides with lower binding energies. For each peptide, 100 separate docking attempts were performed and each docking consisted of 250,000 energy evaluations using the Lamarckian genetic algorithm local search (GALS) algorithm (21). Other docking parameters were all selected based on those described by Csaba and David (22). The selected 50 peptides were further evaluated in a refined search phase with 10 million energy evaluations per docking using the same algorithm. The peptides' lowest docking energies were compared and the 20 lowest ones were selected and sent for synthesis and wet screening. BIACore binding studies indicated that two of the peptides bound to EGFR in a concentration dependant fashion. Especially the peptide with the sequence LARLLT (SEQ ID NO:1) (ranked no. 4 in docking studies) was clearly a potential binder.

The binding pocket selected with the aid of the PSCAN program is shown in Figure 1A (circled). The binding pocket is on a surface easy to be accessed from the outside but not too close to the EGF binding site. We reasoned that the spatial structures in this pocket are less like to vary with or without EGF binding and EGFR dimerization. The pocket has some hydrophobic residues at the bottom and hydrophilic residues around the opening (charged residues: Asp51, Glu73, Glu110, Arg48, Lys56, Arg74; polar residues: Thr106, Ser53, Ser62).

The two peptides we selected to use in this study (LARLLT and control peptide) were docked into the pocket and searched for the best docked structures. The lowest docking energy and free binding energy for LARLLT were -17.9 kcal/mol and -8.54 kcal/mol respectively and those for control peptide were -12.52 kcal/mol and -3.85 kcal/mol.

As shown in Figure 1B, the peptide LARLLT sat nicely inside the pocket, and almost all the residues interacted with residues on EGFR. There were potentially six hydrogen bonds, involving residues at both ends (LEU1, ALA2 and THR6) and in the center (ARG3). At the N-terminal end, the amino group hydrogen of LEU1 interacted with the carboxyl oxygen of GLU73, and the main-chain oxygen of ALA2 interacted with the main-chain amide hydrogen of GLU73. At the carboxyl end, THR6 was both hydrogen bond donor and receptor using its carboxyl-end oxygen to interact with the hydroxyl hydrogen of SER53 and its side-chain hydroxyl hydrogen to interact with the carboxyl oxygen of ASP51. In the middle, ARG3 interacted with EGFR through both electrostatic interaction (with GLU73 and GLU110) and hydrogen bonding. After these three nicely spaced key residues (LEU1, THR6, ARG3) fixing into the EGFR pocket like anchors, they also facilitated the interaction between LARLLT's more hydrophobic residues ALA2, LEU4 and LEU5 with the more hydrophobic bottom in the pocket. The detailed interactions between the residues on LARLLT and EGFR as shown in the docking structure (Figure 1) were in nice agreement with the M-I pairing theory (20).

In contrast, as shown in Figure 1C, the control peptide SEQ ID NO:2, with the same amino acid composition but scrambled sequence, could not even fit inside the pocket. The charged ARG1 interacted with ASP51, but other interactions were just too weak to hold down control peptide.

### EXAMPLE 2

The peptide LARLLT was synthesized and purified by GL Biochem Ltd. (Shanghai), and the structure and purity were all confirmed by HPLC and MS. For comparison, a peptide designated D4* with the same amino acids composition as LARLLT but scrambled sequences (RTALLL, control peptide (SEQ ID NO:2)) was also synthesized. To fluorescently label the two peptides, Cy5.5-NHS was mixed at a 1:2 molar ratio with either LARLLT or the control peptide, and were incubated at 25°C over night, protected from light. These fluorescent peptideswere used without further purification.

### EXAMPLE 3

EGF or LARLLT/control peptides were dissolved in PBS-EDTA and mixed with N-Succinimidyl 3-(2-pyridyldithio) propionate (SPDP) dissolved in DMSO at 1:1.2 molar ratio for 1 hour in room temperature and then lyophilized. In the mean time, DSPE-PEG2000-Mal lipids in chloroform were dried into a thin film and hydrated in HEPES buffer (pH 7.4) to about 0.4 mM concentration. For conjugation, the thioated protein or peptides were added to tris(2-carboxyethyl) phosphine (TCEP) solutions, incubated for 1 hour at room temperature under nitrogen, quickly mixed with the MAL-PEG2000-DSPE micelle solution, and reacted while maintaining stirring under nitrogen at 10°C overnight at 5:1 molar ratio. By HPLC analysis, almost all the Mal-PEG-DSPEs were modified after such reactions. The excess protein/peptides may be removed using standard techniques, for example, by gel filtration column, dialysis etc.

### EXAMPLE 4

Liposomes were all prepared using the dry film hydration method, followed by extrusion several times through 100nm membranes. Most liposomes used in this study started with the lipid composition of EPC:CHOL:DSPE-PEG =10:5:0.5. Except for rhodamine or fluorescein labeled liposomes, either rhodamine-DHPE or fluorescein-DHPE was added to the lipid composition at about 0.6% mol. total lipids.

For Cy5.5 labeled liposomes, Cy5.5-DSPE was synthesized first by mixing Cy5.5-NHS, DSPE and triethylamine at 3:1:3.5 molar ratio in chloroform and incubating (protected from light) at 25°C over night. The resulting Cy5.5-DSPE was incorporated into the lipid composition at about 0.5% mol. total lipids. The excess Cy5.5 was removed after liposome formation by dialysis.

### EXAMPLE 5

Liposomes encapsulating doxorubicin were made following the transmembrane gradient procedure developed (23, 24). Briefly, lipids were hydrated in 125 mM ammonium sulfate at pH 4.0, and extrude to obtain ~100nm liposomes. They were then dialyzed in HEPES buffer (pH 7.5) and diluted to the required concentration with 20mM HEPES, 150nM NaCl (pH 7.5). Doxorubicin was added and incubated with the liposomes at 60°C for 10 min.

The ligand conjugated lipids were inserted into the preformed liposomes based on the procedure developed by Ishida et al. with minor modifications (25). Briefly, DSPE-PEG2000-ligand micelle solutions were added to the preformed liposome solutions at 9:100 mol. lipid ratios and incubated at 60°C for 1 hour (55°C and 30min. for doxorubicin containing liposomes). The solutions were then dialyzed against PBS using SnakeSkinTM Pleated Dialysis Tubing 10,000 MWCO (Pierce Chemical Company) for 4 hours. As control, mPEG2000-DSPE micelles were also mixed and incubated at the same ratio to make non-targeted liposomes.

The peptide ligands were conjugated to DSPE-PEG2000 by a sulfhydryl-maleimide coupling reaction. The reactions were monitored and confirmed using Reverse Phase HPLC analysis. The resulted peptide-PEG-DSPE or the mPEG-DSPE controls were incorporated into preformed liposomes using widely used incubation methods (25). We tested different peptide-PEG-lipid (or mPEG-lipid) to lipid ratios (3:100, 6:100, and 9:100 mol.) during the incubation and selected the 9:100 ratio to deter nonspecific binding of the liposomes to most cells. We confirmed that such a procedure was not disturbing to the integrity of the liposomes. No more than 5% of the encapsulated doxorubicin leaked during and after the procedure. The final size distribution of the liposomes were determined by photon correlation spectroscopy (PCS) using a Zetasizer3000H (Malvern Instruments). Compared to the liposomes made right after the extrusion (~110nm), the particle sizes after the peptide lipid incorporation were slightly bigger (130~150nm) but still stable.

### EXAMPLE 6

The human non-small cell lung carcinoma cell line H1299 and the human lung adenocarcinoma cell line SPCA1, both have high EGFR expressing levels, were used in this study. The cells were cultured in RPMI1640 culture medium containing 10% fetal bovine serum at 4°C in humidified atmosphere containing 5% CO2.

Both H1299 and SPCA1 xenograft mouse models were prepared by the animal experimental center of Shanghai Cancer Institute. They were used in the in vivo imaging experiments about 3-4 weeks after tumor cell inoculation (tumor size about 5-7mm diameter), and humanely sacrificed afterwards. The animal study protocols were approved by the Animal Study Committee of Shanghai Jiaotong University, School of Pharmacy.

### EXAMPLE 7

EGFR high-expressing H1299 or SPCA1 cells were plated in 35mm-diameter culture dish (1×106cells per dish) and cultured in RPMI1640 medium for 24-48 hours. After the cell culture reached about 80% confluence, ligand-conjugated rhodamine liposomes were diluted in RPMI1640 and added into the culture dish at either 4°C or 37°C. In the competitive binding experiments, 50 fold molar excess of free LARLLT or EGF were added into the medium 2 hours before the addition of LARLLT incorporated liposomes. The cells were incubated at the specific temperatures for 4 hours, and then washed six times with PBS (pH 7.4). The remaining bound and internalized fluorescence lipid were visualized using a Confocal Laser scanning Microscope (CLSM, Zeiss LSM, Germany). For the flow cytometric studies, H1299 or SPCA1 cells were plated in 35mm-diameter well until cells grew up to about 80% confluence. After incubation with ligand-modified fluorescein labeled liposomes for 3 hours at 37°C, cells were washed with PBS, treated with trypsin for suspension, and analyzed by a flow cytometer (BD FACSCalibur, Becton Dickinson).

The EGF, LARLLT, or control peptide modified rhodamine labeled liposomes were tested for their binding ability to EGFR high-expressing cells in vitro. As shown in Figure 2A, both EGF and LARLLT modified liposomes bound extensively to the H1299 cells. The fluorescence is a bit stronger with EGF modified liposomes than LARLLT modified liposomes, suggesting tighter binding by EGF. But with control peptide, there was almost no fluorescence shown. Similar situations were observed with another EGFR expressing cell line SPC-A1 (Figure 2C)

The detailed binding characteristics were further evaluated in H1299 cells at different temperatures. At 4°, the fluorescence was mostly seen on cell surfaces, and could be largely competed off by excess unlabeled free LARLLT (Figure 2B-iii & iv). At 37°, however, the competition effect of free LARLLT was less obvious (Figure. 2B-I & ii), suggesting there might be active endocytosis and receptor turnaround after the binding of LARLLT to EGFR. It is also interesting to note that the presence of free EGF at 37° actually affected the LARLLT liposome fluorescence to a certain degree, although not completely (Figure. 2B-v &vi). It is possible that EGF interfered with the endocytic pathway of LARLLT after binding.

Figure 3 showed the LARLLT modified liposome binding and uptake by H1299 cells in higher magnification (Figure 3A) and in Z stack serial scan images (Figure 3B). Lipid fluorescence was seen in numerous endocytic vesicles inside cells. It is noteworthy that the binding of LARLLT to a trivial surface pocket on EGFR far away from the EGF binding site can initiate such active endocytic activities.

In order to compare the different binding efficiencies of LARLLT and control peptide incorporated liposomes in larger cell populations, we used flow cytometry. Liposomes were labeled with fluorescein-DSPE and added to H1299 and SPCA1 cells respectively. After 3 hours of incubation, 75% of H1299 cells were highly fluorescent (101~103 FL1 value) with LARLLT liposome binding, but only 27% were fluorescent after control peptide liposomes binding. For SPCA1 cells, 68% were fluorescent (35~103 FL1 value gate) with LARLLT liposome and 17% with control peptide lipsomes.

### EXAMPLE 8

H1299 and SPCA1 cells were plated at the density of 10⁴cells/well in 96-well plates and grown overnight. Free doxorubicin, LARLLT modified doxorubicin liposomes and control peptide modified doxorubicin liposomes were added into the medium and incubated for 2 hours. The cells were then washed with fresh medium, and let grown for another 48 hours. The cell viabilities after different treatments were determined using a MTT assay (26).

Doxorubicin loaded liposomes were modified by LARLLT or control peptide conjugated lipids and added to EGFR high expressing cells. The cell killing effects were assayed using MTT. In both H1299 and SPCA1 cells, the LARLLT modified liposomes were better in delivering drugs than control peptide modified liposomes (Figure 4A&B). The calculated IC₅₀s for LARLLT liposomes were about 3-5 times cytotoxic than those for control peptide liposomes in both cases.

### EXAMPLE 9

Cy5.5, LARLLT-Cy5.5, control peptide-Cy5.5, and LARLLT and control peptide incorporated Cy5.5 labeled liposomes were injected through the tail vein to H1299 or SPCA1 xenograft tumor bearing mice. At various time points afterwards, the mice were anesthetized and imaged with an Optix in vivo fluorescence imaging system (GE Healthcare). Excepted for the Cy5.5 dye only control, all the other groups contained at least 3 mice. The representative images from the same most representative mouse in each group were shown. The images had been processed using the fluorescence lifetime gating for Cy5.5 (0.8-1.1ns) to remove most autofluorescent interferences.

LARLLT and control peptide were labeled using the near infrared dye Cy5.5 and injected via tail vein to H1299 tumor bearing mice. Cy5.5 fluorescence distributions inside the mice were imaged at various time points after injection. In all groups including the free dye control, fluorescences were first seen all over the body, and then quickly afterwards in the kidney and bladder. To avoid the interference from the strong fluorescence in kidney and bladder, we only showed the focused imaging scan at the subcutaneous tumor bearing region (Figure 5), In Cy5.5 dye injected mice, the fluorescence signal in the tumor region was the same as background. But in LARLLT-Cy5.5 injected mice, between 1 to 6 hours after injection, fluorescence signals in the tumor tissue were much stronger, as compared to the surrounding tissues. In contrast, Cy5.5-control peptide injected mice didn't show any fluorescence accumulation in the tumor region either.

LARLLT and control peptide modified Cy5.5 labeled liposomes were injected via the tail vein into H1299 tumor bearing mice. The entire bodies of the mice were scanned at various times after the injection and the fluorescence intensity from the tumor bearing region at different time points were quantified. As shown in Figure 6, LARLLT modified liposomes accumulated gradually inside the tumor region from 6 hours and on. Even after 80 hours, the signals and the contrast were still significant. But for control peptide modified liposomes, the fluorescence intensities in the tumor region were always as background. We calculated the relative ratios of fluorescence intensities inside the tumor region to the whole body signals (Table 1). Fluorescence intensities(NC) of tumor region and the whole body were calculated using eXplore OptiView 1.0.0.0 software. The percentage of the fluorescence inside the tumor region were also listed.

Table 1 Fluorescence intensities in tumor region at various time points after fluorescent liposome injection.

| | Body | | Tumor | | Tumor / Body | |
|---|---|---|---|---|---|---|
| | LARLLT-L | control peptide-L | LARLLT-L | control peptide-L | LARLLT-L | control peptide-L |
| 1hour | 4.68E+04 | 4.15E+04 | 6.00E+03 | 4.16E+03 | 13% | 10% |
| 6hour | 2.75E+04 | 2.58E+04 | 4.37E+03 | 2.45E+03 | 16% | 13% |
| 12hour | 2.34E+04 | 1.91E-04 | 3.92E+03 | 1.99E+03 | 17% | 10% |
| 24hour | 1.84E+04 | 1.99E+04 | 3.38E+03 | 2.02E+03 | 18% | 11% |
| 48hour | 1.34E+04 | 1.30E+04 | 2.69E+03 | 1.35E+03 | 20% | 10% |
| 80hour | 1.07E+04 | 9.92E+03 | 1.99E+03 | 1.14E+03 | 19% | 11% |

The ratios actually got better and better from 1 hour after injection all the way to 76-80 hours, when it seemed to plateau, suggesting a gradual accumulation and the targeted liposomes inside the tumors and strong retention afterwards.

In some embodiments, peptides of the invention may be designed to bind to a surface pocket away from the EGF binding site. This may allow peptides of the invention (and therapeutics attached to the peptides) to bind EGFR expressing cells even if the binding affinities of the peptides may be less than the binding affinity of EGF since there will be no direct competition for binding between the peptides and EGF.

When peptides of the invention are conjugated to various compounds, for example, to the distal end of PEG2000-DSPE and incorporated into liposomes, peptides of the invention retain their EGFR binding ability and mediate specific attachment and uptake of the compound (e.g., liposomes) by EGFR high-expressing cells. As shown above, when compounds to which the peptides of the invention are attached (e.g., the peptide incorporated liposomes shown above) were injected in vivo, the compounds are directed to the target cells (e.g., tumor cells) and cause the peptide conjugated compound (e.g., peptide conjugated liposomes) to accumulate at the target (e.g., in the tumor).

In our studies, we used only PEG2000 linker, and in vitro binding to cells was found most significant (with minimal background nonspecific, binding) when the ligand containing lipids were incorporated under the condition of 2-4:100 lipid ratio. Any other linker known to those skilled in the art may be used. Likewise other ratios of lipids may be used.

The same liposome formulations were used in vivo. The liposomes had relative high surface ligand-PEG densities and slowly accumulated in tumors. In fact, several earlier studies have indicated that during circulation and extravasation, active targeting liposomes would at the best behave similar as Stealth liposomes (4, 6, 41). Their targeting advantages were only significant after extravasation into the tumor tissue, when they could bind tightly with cancer cells and facilitate cell uptake (8, 42). Our observations using the in vivo fluorescence imaging system (Figure 6) agreed with such a mechanism. The ligand incorporated LARLLT liposomes were found to stay in the tumor tissue for a very long time (>80 hours). The control group, however, didn't show significant accumulation in the tumor tissue around 12-24 hour points, as we would expect for non-targeting stealth liposomes.

Small animal in vivo fluorescence imaging is a newly developed tool for monitoring the biodistribution of labeled drugs and delivery systems. Cy5.5 is the most commonly used dye because of its superior tissue penetration and low background. The main advantage of the in vivo imaging technique is that it's noninvasive and allows continues monitoring of the same live animals throughout the course of the study. Many earlier studies had used radio labeled drugs or lipids to follow their distribution in vivo, it was necessary to sacrifice numbers of animals at different time points for obtain averaged readouts. In our experiences using in vivo imaging system, different animals in a study group may still have variation in their general health, physiology, metabolism, tumor size and location etc. The time-sequenced distribution profile of active targeting liposomes also agreed well with what had been reported using other methods (8, 41).

### EXAMPLE 10

The ability of liposomes targeted using the peptides of the invention to enhance accumulation of drug in the target tumor was assessed. 5mg/kg doxorubicin was injected intravenously into H460 tumor bearing nude mice. Free doxorubicin (Figure 7, Dox) was compared to doxorubicin incorporated into a PEGylated liposome (Figure 7, PEG) and doxorubicin incorporated into a liposome comprising a peptide of the invention (Figure 7, D4). Mice were sacrificed at 0.5h, 6h and 48h post-injection and the tumors were excised and assayed for doxorubicin content. N=3 animals were used per experimental group.

As shown in Figure 7, liposomes comprising the peptides invention resulted in significantly more doxorubicin being accumulated in the tumor as compared to free doxorubicin and doxorubicin in a PEGylated liposome.

### EXAMPLE 11

FACS analysis of binding of liposomes comprising peptides of the invention

H-460 cells were grown in cell culture plate under sterile conditions and harvested by scraping. The liposome samples (empty Thermodox liposome, ThermoDox, D4-ThermoDox, D4*-ThermoDox where D4 ThermoDox comprises SEQ ID NO:1 and D4*ThermoDox comprises SEQ ID NO:2) and doxorubicin hydrochloride were added and incubated at 37°C with H-460 cells for 4 hours. Two different liposomes comprising different amounts of the peptide of the invention (D4-ThermoDox) were tested. The two liposomes were designated D4-ThermoDox2+2, which comprised DPPC:MSPC: DSPE-MPEG2000:D4-DSPE-MPEG2000 in a 90:10:2:2 molar ratio and D4-ThermoDox4+4, which comprised DPPC:MSPC: DSPE-MPEG2000:D4-DSPE-MPEG2000 in a 90:10:4:4 molar ratio. The D4*-ThermoDox comprised DPPC:MSPC: DSPE-MPEG2000:D4*-DSPE-MPEG2000 in a 90:10:2:2 molar ratio. After incubation, the cells were washed 4 times with PBS, and counted by FACS using doxorubicin fluorescence (excitation at 488nm, and emission at 575nm).

The following groups were examined: a. Non-treatment control; b. ThermoDox at approx. 0.01-10 µg (doxorubicin)/mL; c. D4-ThermoDox2+2 at approx. 0.01-10 µg (doxorubicin)/mL; d. D4-ThermoDox4+4 at approx. 0.01-10 µg (doxorubicin)/mL; e. D4*-ThermoDox at approx. 0.01-10 µg (doxorubicin)/mL; and f. doxorubicin hydrochloride 0.01-10 µg.

The experiments were repeated at least three times, and the representative histograms from the same experiments are shown. The detailed numbers may shift due to the variations in cell number and FACS parameters, but the general pattern and formulation differences were always consistent.

At the 10 µg/ml dose, the doxorubicin fluorescence intensities of D4-ThermoDox(2+2) in vitro are stronger than ThermoDox and D4*-ThermoDox, only weaker than free doxorubicin hydrochloride. Given that the free drug is relatively more permeable through cell membrane, the D4-liposme is confirmed to bind more to H460 cells than the control liposomes. At the 1µg/ml dose, the D4-ThermoDox (2+2, 4+4) treated cells generated more doxorubicin fluorescence signal than ThermoDox and D4*-ThermoDox. At both the 0.1 µg/ml and 0.01 µg/ml doses, there were no significant differences between liposomes of different formulations

Figure 8 is a line graph of the mean of each of the FACS histograms plotted against the amount of doxorubicin administered for each formulations tested. The doxorubicin uptake (mean fluorescence intensity) was the highest using the drug solution because it can permeate cell membrane quite efficiently. Comparing the different liposome formulations, the two D4 liposome preparations were much better than Thermodox controls and D4* liposome controls.

References:

1. Gabizon A, Catane R, Uziely B, et al. Prolonged circulation time and enhanced accumulation in malignant exudates of doxorubicin encapsulated in polyethylene-glycol coated liposomes. Cancer Res 1994;54(4):987-92.

2. Maruyama K, Takizawa T, Yuda T, Kennel SJ, Huang L, Iwatsuru M. Targetability of novel immunoliposomes modified with amphipathic poly(ethylene glycol)s conjugated at their distal terminals to monoclonal antibodies. Biochim Biophys Acta 1995;1234(1):74-80.

3. Park JW, Hong K, Carter P, et al. Development of anti-p185HER2 immunoliposomes for cancer therapy. Proc Natl Acad Sci U S A 1995;92(5):1327-31.

4. Goren D, Horowitz AT, Zalipsky S, Woodle MC, Yarden Y, Gabizon A. Targeting of stealth liposomes to erbB-2 (Her/2) receptor: in vitro and in vivo studies. Br J Cancer 1996;74:1749-1756.

5. Lopes de Menezes DE, Pilarski LM, Allen TM. In vitro and in vivo targeting of immunoliposomal doxorubicin to human B-cell lymphoma. Cancer Res 1998;58(15):3320-30.

6. Matsumura Y, Gotoh M, Muro K, et al. Phase I and pharmacokinetic study of MCC-465, a doxorubicin (DXR) encapsulated in PEG immunoliposome, in patients with metastatic stomach cancer. Ann Oncol 2004;15(3):517-25.

7. Lee RJ, Low PS. Folate-mediated tumor cell targeting of liposome-entrapped doxorubicin in vitro. Biochim Biophys Acta 1995;1233(2):134-44.

8. Gabizon A, Horowitz AT, Goren D, TzemachD, Shmeeda H, Zalipsky S. In vivo fate of folate-targeted polyethylene-glycol liposomes in tumor-bearing mice. Clin cancer Res 2003;9:6551-9.

9. Terada T, Mizobata M, Kawakami S, Yabe Y, Yamashita F, Hashida M. Basic fibroblast growth factor-binding peptide as a novel targeting ligand of drug carrier to tumor cells. J Drug Target 2006;14(8):536-45.

10. Schiffelers RM, Koning GA, ten Hagen TL, et al. Anti-tumor efficacy of tumor vasculature-targeted liposomal doxorubicin. J Control Release 2003;91(1-2):115-22.

11. Salomon DS, Brandt R, Ciardiello F, Normanno N. Epidermal growth factor-related peptides and their receptors in human malignancies. Crit Rev Oncol Hematol 1995; 19:183-232.

12. Lutsenko SV, Feldman NB, Severin SE. Cytotoxic and antitumor activities of doxorubicin conjugates with the epidermal growth factor and its receptor-binding fragment. J Drug Target 2002;10(7):567-71.

13. Jinno H, Ueda M, Ozawa S, et al. Epidermal growth factor receptor-dependent cytotoxic effect by an EGF-ribonuclease conjugate on human cancer cell lines--a trial for less immunogenic chimeric toxin. Cancer Chemother Pharmacol 1996;38(4):303-8.

14. Kullberg EB, Nestor M, Gedda L. Tumor-cell targeted epiderimal growth factor liposomes loaded with boronated acridine: uptake and processing. Pharm Res 2003; 20(2):229-36.

15. Chen P, Mrkobrada M, Vallis KA, et al. Comparative antiproliferative effects of (111) In-DTPA-hEGF, chemotherapeutic agents and gamma-radiation on EGFR-positive breast cancer cells. Nucl Med Biol 2002;29(6):693-9.

16. Blessing T, Kursa M, Holzhauser R, Kircheis R, Wagner E. Different strategies for formation of pegylated EGF-conjugated PEI/DNA complexes for targeted gene delivery. Bioconjug Chem 2001;12(4):529-37.

17. Mamot C, Drummond DC, Greiser U, et al. Epidermal growth factor receptor (EGFR)-targeted immunoliposomes mediate specific and efficient drug delivery to EGFR-and EGFRvIII-overexpressing tumor cells. Cancer Res 2003;63(12):3154-61.

18. Mamot C, Drummond DC, Noble CO, et al. Epidermal growth factor receptor-targeted immunoliposomes significantly enhance the efficacy of multiple anticancer drugs in vivo. Cancer Res 2005; 65(24):11631-8.

19. Mamot C, Ritschard R, Kung W, Park JW, Herrmann R, Rochlitz CF. EGFR-targeted immunoliposomes derived from the monoclonal antibody EMD72000 mediate specific and efficient drug delivery to a variety of colorectal cancer cells. J Drug Target 2006; 14(4):215-23.

20. Heal JR, Roberts GW, Raynes JG, Bhakoo A, Miller AD. Specific interactions between sense and complementary peptides: the basis for the proteomic code. Chembiochem 2002;3(2-3):136.-51.

21. Morris GM, Goodsell DS, Halliday RS, et al. Automated docking using a Lamarckian genetic algorithm and an empirical binding free energy function. J Comput Chem 1998;19:1639-62.

22. Hetenyi C. van der Spoel D. Efficient docking of peptides to proteins without prior knowledge of the binding site. Protein Sci 2002;11(7):1729-37.

23. Mayer LD, Tai LC, Bally MB, Mitilenes GN, Ginsberg RS, Cullis PR. Characterization of liposomal systems containing doxorubicin entrapped in response to pH gradients. Biochim Biophys Acta 1990;1025(2):143-51.

24. Haran G, Cohen R, Bar LK, Barenholz Y. Transmembrane ammonium sulfate gradients in liposomes produce efficient and stable entrapment of amphipathic weak bases. Biochim Biophys Acta 1993;1151(2):201-15. Erratum in: Biochim Biophys Acta 1994;1190(1):197.

25. Ishida T, Iden DL, Allen TM. A combinatorial approach to producing sterically stabilized (Stealth) immunoliposomal drugs. FEBS Lett 1999;460 (1):129-33.

26. Scudiero DA, Shoemaker RH, Paull KD, et al. Evaluation of a soluble tetrazolium/formazan assay for cell growth and drug sensitivity in culture using human and other tumor cell lines. Cancer Res 1988;48(17):4827-33.

27. Schmidt M, Vakalopoulou E, Schneider DW, Wels W. Construction and functional characterization of scFv(14E1)-ETA -a novel, highly potent antibody-toxin specific for the EGF receptor. Br J Cancer 1997; 75(11):1575-84.

28. Liu X, Tian P, Yu Y, Yao M, Cao X, Gu J. Enhanced antitumor effect of EGF R-targeted p21WAF-1 and GM-CSF gene transfer in the established murine hepatoma by peritumoral injection. Cancer Gene Ther 2002;9(1):100-8.

29. Li Z, Zhao R, Wu X, et al. Identification and characterization of a novel peptide ligand of epidermal growth factor receptor for targeted delivery of therapeutics. FASEB J 2005;19(14):1978-85.

30. de Graaf C, Pospisil P, Pos W, Folkers G, Vermeulen NP. Binding mode prediction of cytochrome p450 and thymidine kinase protein-ligand complexes by consideration of water and rescoring in automated docking. J Med Chem 2005;48(7):2308-18.

31. Rogers JP, Beuscher AE 4th, Flajolet M, et al.. Discovery of protein phosphatase 2C inhibitors by virtual screening. J Med Chem 2006; 49(5):1658-67.

32. Bhakoo A, Raynes JG, Heal JR, Keller M, Miller AD. De-novo design of complementary (antisense) peptide mini-receptor inhibitor of interleukin 18 (IL-18). Mol Immunol 2004;41(12):1217-24.

33. Mastrobattista E, Koning GA, Storm G. Immunoliposomes for the targeted delivery of antitumor drugs. Adv Drug Deliv Rev 1999;40(1-2):103-127.

34. Gabizon AA, Shmeeda H, Zalipsky S. Pros and cons of the liposome platform in cancer drug targeting. J Liposome Res 2006;16(3):175-83.

35. Koning GA, Morselt HW, Gorter A, et al. Pharmacokinetics of differently designed immunoliposome formulations in rats with or without hepatic colon cancer metastases. Pharm Res 2001;18(9):1291-8.

36. Koning GA, Morselt HW, Gorter A, et al. Interaction of differently designed immunoliposomes with colon cancer cells and Kupffer cells. An in vitro comparison. Pharm Res 2003;20(8):1249-57.

37. Derksen JT, Morselt HW, Scherphof GL. Uptake and processing of immunoglobulin-coated liposomes by subpopulations of rat liver macrophages. Biochim Biophys Acta 1988;971(2):127-36.

38. Maruyama K, Takahashi N, Tagawa T, Nagaike K, Iwatsuru M. Immunoliposomes bearing poloethylene glycol-coupled Fab 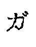 fragment show prolonged circulation time and high extravasation into targeted solid tumors in vivo. FEBS Lett 1997;413(1):177-80.

39. Huwyler J, Yang J, Pardridge WM. Receptor mediated delivery of daunomycin using immunoliposomes: pharmacokinetics and tissue distribution in the rat. J Pharmacol Exp Ther 1997; 282(3):1541-6.

40. Tardi P, Bally MB, Harasym TO. Clearance properties of liposomes involving conjugated proteins for targeting. Adv Drug Deliv Rev 1998;32(1-2):99-118.

41. Sapra P, Moase EH, Ma J, Allen TM. Improved therapeutic responses in a xenograft model of human B lymphoma (Namalwa) for liposomal vineristine versus liposomal doxorubicin targeted via anti-CD 19 IgG2a or Fab' fragments. Clin Cancer Res 2004;10(3):1100-11.

42. Kirpotin DB, Drummond DC, Shao Y, et al. Antibody targeting of long-circulating lipidic nanoparticles does not increase tumor localization but does increase internalization in animal models. Cancer Res 2006;66(13):6732-40.

All publications, patents and patent applications mentioned in this specification are indicative of the level of skill of those skilled in the art to which this invention pertains.

## Claims

1. A peptide comprising a sequence selected from the group consisting of SEQ ID NO:1, SEQ ID NO:3, SEQ ID NO:4, SEQ ID NO:5, SEQ ID NO:6, SEQ ID NO:7, and SEQ ID NO:8 wherein the peptide is from 6 to 15 amino acids in length, and wherein Xaa₆ of SEQ ID NO: 8 is selected from the group consisting of serine, asparagine and cysteine.

2. A peptide according to claim 1, comprising SEQ ID NO: 1 or consisting of SEQ ID NO:1.

3. A molecule comprising a lipid associated with a peptide according to claim 1 or 2.

4. A molecule according to claim 3, wherein
(A) the molecule comprises a linker between the lipid and the peptide, optionally wherein the lipid is 1,2-Distearoyl-sn-Glycero-3-Phosphoethanolamine (DSPE) or wherein the linker comprises polyethylene glycol (PEG); or
(B) the molecule comprises 1,2-Distearoyl-sn-Glycero-3-Phosphoethanolamine-N-[Methoxy(Polyethylene glycol)-2000] (DSPE-PEG2000); or
(C) the molecule comprises SEQ ID NO:1.

5. A liposome comprising:
a molecule comprising a lipid associated with a peptide according to claim 1 or 2.

6. A liposome according to claim 5, wherein
(A) the molecule comprises a linker between the lipid and the peptide; or
(B) the lipid is 1,2-Distearoyl-sn-Glycero-3-Phosphoethanolamine (DSPE), optionally wherein the linker comprises polyethylene glycol (PEG) or wherein the liposome comprises 1,2-Distearoyl-sn-Glycero-3-Phosphoethanolamine-N-[Methoxy(Polyethylene glycol)-2000] (DSPE-PEG2000)-LARLLT; or
(C) the liposome further comprises a lipid selected from the group consisting of phosphatidylcholines, phosphatidylglycerols, and lysolipids; or
(D) the liposome comprises 1,2-Dipalmitoyl-sn-glycero-3-phosphocholine (DPPC) and monostearoylphosphatidylcholine (MSPC); or
(E) the liposome further comprises a compound selected from the group consisting of therapeutic compounds and imaging compounds, optionally
(i) wherein the liposome comprises a bilayer and defines an interior space and the compound is in the interior space of the liposome, in the bilayer of the liposome, or is in both the interior space and bilayer; or
(ii) wherein the therapeutic compound is an anticancer compound optionally wherein the anticancer compound is selected from a group consisting of alkylating agents, antimetabolites , spindle poison plant alkaloids, cytotoxic antitumor antibiotics, topoisomerase inhibitors, monoclonal antibodies or fragments thereof, photosensitizers, kinase inhibitors, antitumor enzymes and inhibitors of enzymes, apoptosis-inducers, biological response modifiers, anti-hormones, retinoids and platinum containing compounds or wherein the anticancer compound is selected from a group consisting of docetaxel, doxorubicin, and carboplatin; or
(iii) wherein the liposome comprises an imaging agent; or
(iv) wherein the liposome comprises 1,2-Distearoyl-sn-Glycero-3-Phosphoethanolamine-N-[Methoxy(Polyethylene glycol)-2000] (DSPE-PEG2000)-LARLLT, wherein the therapeutic agent is carboplatin.

7. A compound for imaging associated with a peptide according to claim 1 or 2.

8. A compound according to claim 7, wherein
(A) the peptide is attached to a lipid and the peptide-attached lipid is in the form of a liposome; or
(B) the peptide-attached lipid comprises a linker between the lipid and the peptide, optionally;
(i) wherein the lipid is 1,2-Distearoyl-sn-Glycero-3-Phosphoethanolamine (DSPE), optionally wherein the linker comprises polyethylene glycol (PEG); or
(ii) wherein the peptide-attached lipid comprises 1,2-Distearoyl-sn-Glycero-3-Phosphoethanolamine-N-[Methoxy(Polyethylene glycol)-2000] (DSPE-PEG2000)-LARLLT; or
(C) the liposome further comprises 1,2-Dipalmitoyl-sn-glycero-3-phosphocholine (DPPC) and monostearoylphosphatidylcholine (MSPC) and wherein the liposome has a gel to liquid phase transition temperature of from about 39.0°C to about 45°C; or
(D) the liposome further comprises a PEGylated lipid; or
(E) the liposome comprises a bilayer and defines an interior space and the compound is in the interior space of the liposome, in the bilayer of the liposome or is in both the interior space and bilayer.

9. A liposome for use in treating cancer in a subject in need thereof, wherein the liposome comprises a lipid associated with a peptide according to claim 1 or 2, and the liposome comprises an anticancer compound.

10. A liposome for use according to claim 9, wherein the peptide is attached to the lipid, optionally wherein the peptide-attached lipid comprises a linker between the lipid and the peptide, or wherein the lipid is 1,2-Distearoyl-sn-Glycero-3-Phosphoethanolamine (DSPE), or wherein the linker comprises polyethylene glycol (PEG), or wherein the peptide-attached lipid comprises 1,2-Distearoyl-sn-Glycero-3-Phosphoethanolamine-N-[Methoxy(Polyethylene glycol)-2000] (DSPE-PEG2000)-LARLLT.

11. A liposome for use according to claim 9, wherein the liposome further comprises 1,2-Dipalmitoyl-sn-glycero-3-phosphocholine (DPPC) and monostearoylphosphatidylcholine (MSPC) and wherein the liposome has a gel to liquid phase transition temperature of from about 39.0°C to about 454°C, or wherein the liposome comprises a PEGylated lipid, or wherein the liposome comprises a bilayer and defines an interior space and the anticancer compound is in the interior space of the liposome, in the bilayer of the liposome or is in both the interior space and bilayer, or wherein the anticancer compound is selected from a group consisting of alkylating agents, antimetabolites, spindle poison plant alkaloids, cytotoxic antitumor antibiotics, topoisomerase inhibitors, monoclonal antibodies or fragments thereof, photosensitizers, kinase inhibitors, antitumor enzymes and inhibitors of enzymes, apoptosis-inducers, biological response modifiers, anti-hormones, retinoids and platinum containing compounds, or wherein the anticancer compound is selected from a group consisting of docetaxel, doxorubicin, and carboplatin.

12. A liposome for use according to claim 9, wherein the treating comprises heating a portion of the subject, optionally wherein heating comprises application of electromagnetic energy, infrared radiation, or ultrasound.

13. A peptide according to claim 1 or 2, a molecule according to claim 3 or 4, or a liposome according to claim 5 or 6 for use in therapy.

14. A peptide, molecule or liposome according to claim 13, wherein said therapy is treatment of cancer.

## Patentansprüche

1. Peptid mit einer Sequenz, die aus der Gruppe ausgewählt ist, die aus SEQ ID NR.: 1, SEQ ID NR.: 3, SEQ ID NR.: 4, SEQ ID NR.: 5, SEQ ID NR.: 6, SEQ ID NR.: 7 und SEQ ID NR.: 8 besteht, wobei das Peptid 6 bis 15 Aminosäuren lang ist und wobei Xaa₆ von SEQ ID NR.: 8 aus der Gruppe ausgewählt ist, die aus Serin, Asparagin und Cystein besteht.

2. Peptid nach Anspruch 1, das SEQ ID NR.: 1 aufweist oder aus SEQ ID NR.: 1 besteht.

3. Molekül mit einem Lipid, das einem Peptid nach Anspruch 1 oder 2 zugeordnet ist.

4. Molekül nach Anspruch 3, wobei
(A) das Molekül einen Linker zwischen dem Lipid und dem Peptid aufweist, wobei wahlweise das Lipid 1,2-Distearoyl-sn-glycero-3-phosphoethanolamin (DSPE) ist oder wobei der Linker Polyethylenglycol (PEG) aufweist; oder
(B) das Molekül 1,2-Distearoyl-sn-glycero-3-phosphoethanolamin-N-[methoxy(polyethylenglycol)-2000] (DSPE-PEG2000) aufweist; oder
(C) das Molekül SEQ ID NR.: 1 aufweist.

5. Liposom, das aufweist:
ein Molekül mit einem Lipid, das einem Peptid nach Anspruch 1 oder 2 zugeordnet ist.

6. Liposom nach Anspruch 5, wobei
(A) das Molekül einen Linker zwischen dem Lipid und dem Peptid aufweist; oder
(B) das Lipid 1,2-Distearoyl-sn-glycero-3-phosphoethanolamin (DSPE) ist, wobei der Linker wahlweise Polyethylenglycol (PEG) aufweist, oder wobei das Liposom 1,2-Distearoyl-sn-glycero-3-phosphoethanolamin-N-[methoxy(polyethylenglycol)-2000] (DSPE-PEG2000)-LARLLT aufweist; oder
(C) das Liposom außerdem ein Lipid aufweist, das aus der Gruppe ausgewählt ist, die aus Phosphatidylcholinen, Phosphatidylglycerinen und Lysolipiden besteht; oder
(D) das Liposom 1,2-Dipalmitoyl-sn-glycero-3-phosphocholin (DPPC) und Monostearoylphosphatidylcholin (MSPC) aufweist; oder
(E) das Liposom außerdem eine Verbindung aufweist, die aus der Gruppe ausgewählt ist, die aus therapeutischen Verbindungen und Abbildungsverbindungen besteht, wahlweise
(i) wobei das Liposom eine Doppelschicht aufweist und einen Innenraum definiert und die Verbindung sich im Innenraum des Liposoms, in der Doppelschicht des Liposoms oder sowohl im Innenraum als auch in der Doppelschicht befindet; oder
(ii) wobei die therapeutische Verbindung eine Antikrebsverbindung ist, wobei wahlweise die Antikrebsverbindung aus einer Gruppe ausgewählt ist, die aus Alkylierungsmitteln, Antimetaboliten, Spindelgiftpflanzenalkaloiden, zytotoxischen Antitumorantibiotika, Topoisomeraseinhibitoren, monoklonalen Antikörpern oder Fragmenten davon, Photosensibilisatoren, Kinaseinhibitoren, Antitumorenzymen und Inhibitoren von Enzymen, Apoptoseinduktoren, biologischen Reaktionsmodifikatoren, Antihormonen, Retinoiden und Platin enthaltenden Verbindungen besteht, oder wobei die Antikrebsverbindung aus einer Gruppe ausgewählt ist, die aus Docetaxel, Doxorubicin und Carboplatin besteht; oder
(iii) wobei das Liposom ein Abbildungsmittel aufweist; oder
(iv) wobei das Liposom 1,2-Distearoyl-sn-glycero-3-phosphoethanolamin-N-[methoxy(polyethylenglycol)-2000] (DSPE-PEG2000)-LARLLT aufweist, wobei das therapeutische Mittel Carboplatin ist.

7. Verbindung zum Abbilden, die einem Peptid nach Anspruch 1 oder 2 zugeordnet ist.

8. Verbindung nach Anspruch 7, wobei
(A) das Peptid an ein Lipid gebunden ist und das Peptid-gebundene Lipid in Form eines Liposoms vorliegt; oder
(B) das Peptid-gebundene Lipid einen Linker zwischen dem Lipid und dem Peptid aufweist, wahlweise
(i) wobei das Lipid 1,2-Distearoyl-sn-glycero-3-phosphoethanolamin (DSPE) ist, wobei wahlweise der Linker Polyethylenglycol (PEG) aufweist; oder
(ii) wobei das Peptid-gebundene Lipid 1,2-Distearoyl-sn-glycero-3-phosphoethanolamin-N-[methoxy(polyethylenglycol)-2000] (DSPE-PEG2000)-LARLLT aufweist; oder
(C) das Liposom außerdem 1,2-Dipalmitoyl-sn-glycero-3-phosphocholin (DPPC) und Monostearoylphosphatidylcholin (MSPC) aufweist und wobei das Liposom eine Gel-Flüssigphasen-Übergangstemperatur von etwa 39,0 °C bis etwa 45 °C aufweist; oder
(D) das Liposom außerdem ein PEGyliertes Lipid aufweist; oder
(E) das Liposom eine Doppelschicht aufweist und einen Innenraum definiert und die Verbindung sich im Innenraum des Liposoms, in der Doppelschicht des Liposoms oder sowohl im Innenraum als auch in der Doppelschicht befindet.

9. Liposom zur Verwendung bei der Behandlung von Krebs bei einer Person, die diese benötigt, wobei das Liposom ein Lipid aufweist, das einem Peptid nach Anspruch 1 oder 2 zugeordnet ist, und das Liposom eine Antikrebsverbindung aufweist.

10. Liposom zur Verwendung nach Anspruch 9, wobei das Peptid an das Lipid gebunden ist, wobei wahlweise das Peptid-gebundene Lipid einen Linker zwischen dem Lipid und dem Peptid aufweist, oder wobei das Lipid 1,2-Distearoyl-sn-glycero-3-phosphoethanolamin (DSPE) ist oder wobei der Linker Polyethylenglycol (PEG) aufweist, oder wobei das Peptid-gebundene Lipid 1,2-Distearoyl-sn-glycero-3-phosphoethanolamin-N-[methoxy(polyethylenglycol)-2000] (DSPE-PEG2000)-LARLLT aufweist.

11. Liposom zur Verwendung nach Anspruch 9, wobei das Liposom außerdem 1,2-Dipalmitoyl-sn-glycero-3-phosphocholin (DPPC) und Monostearoylphosphatidylcholin (MSPC) aufweist und wobei das Liposom eine Gel-Flüssigphasen-Übergangstemperatur von etwa 39,0 °C bis etwa 45 °C aufweist, oder wobei das Liposom ein PEGyliertes Lipid aufweist, oder wobei das Liposom eine Doppelschicht aufweist und einen Innenraum definiert und die Antikrebsverbindung sich im Innenraum des Liposoms, in der Doppelschicht des Liposoms oder sowohl im Innenraum als auch in der Doppelschicht befindet, oder wobei die Antikrebsverbindung aus einer Gruppe ausgewählt ist, die aus Alkylierungsmitteln, Antimetaboliten, Spindelgiftpflanzenalkaloiden, zytotoxischen Antitumorantibiotika, Topoisomeraseinhibitoren, monoklonalen Antikörpern oder Fragmenten davon, Photosensibilisatoren, Kinaseinhibitoren, Antitumorenzymen und Inhibitoren von Enzymen, Apoptoseinduktoren, biologischen Reaktionsmodifikatoren, Antihormonen, Retinoiden und Platin enthaltenden Verbindungen besteht, oder wobei die Antikrebsverbindung aus einer Gruppe ausgewählt ist, die aus Docetaxel, Doxorubicin und Carboplatin besteht.

12. Liposom zur Verwendung nach Anspruch 9, wobei die Behandlung das Erwärmen eines Teils der Person umfasst, wobei wahlweise das Erwärmen die Anwendung von elektromagnetischer Energie, Infrarotstrahlung oder Ultraschall umfasst.

13. Peptid nach Anspruch 1 oder 2, Molekül nach Anspruch 3 oder 4 oder Liposom nach Anspruch 5 oder 6 zur Verwendung bei einer Therapie.

14. Peptid, Molekül oder Liposom nach Anspruch 13, wobei die Therapie die Behandlung von Krebs ist.

## Revendications

1. Peptide comprenant une séquence choisie dans le groupe comprenant la SEQ ID No. : 1, la SEQ ID No. : 3, la SEQ ID No. : 4, la SEQ ID No. : 5, la SEQ ID No. : 6, la SEQ ID No. : 7, et la SEQ ID No. : 8, dans lequel le peptide a une longueur correspondant à 6 à 15 acides aminés, et dans lequel l'acide aminé Xaa₆ de la SEQ ID No. : 8 est choisi dans le groupe comprenant la sérine, l'asparagine et la cystéine.

2. Peptide selon la revendication 1, comprenant la SEQ ID No. : 1 ou consistant en la SEQ ID No. : 1.

3. Molécule comprenant un lipide associé à un peptide selon la revendication 1 ou 2.

4. Molécule selon la revendication 3, dans laquelle :
(A) la molécule comprend une séquence de liaison entre le lipide et le peptide dans laquelle, éventuellement, le lipide est la 1,2-distéaroyl-sn-glycéro-3-phosphoéthanolamine (DSPE) ou dans laquelle la séquence de liaison comprend du polyéthylène glycol (PEG) ; ou
(B) la molécule comprend le 1,2-distéaroyl-sn-glycéro-3-phosphoéthanolamine-N-[méthoxy(polyéthylène glycol)-2000] (DSPE-PEG2000) ; ou
(C) la molécule comprend la SEQ ID No. : 1.

5. Liposome comprenant
une molécule comprenant un lipide associé à un peptide selon la revendication 1 ou 2.

6. Liposome selon la revendication 5, dans lequel :
(A) la molécule comprend une séquence de liaison entre le lipide et le peptide ; ou
(B) le lipide est la 1,2-distéaroyl-sn-glycéro-3-phosphoéthanolamine (DSPE) dans lequel, éventuellement, la séquence de liaison comprend du polyéthylène glycol (PEG), ou dans lequel le liposome comprend la 1,2-distéaroyl-sn-glycéro-3-phosphoéthanolamine-N-[méthoxy(polyéthylène glycol)-2000] (DSPE-PEG2000)-LARLLT ; ou
(C) le liposome comprend en outre un lipide choisi dans le groupe comprenant des phosphatidylcholines, des phosphatidylglycérols, et des lysolipides ; ou
(D) le liposome comprend la 1,2-dipalmitoyl-sn-glycéro-3-phosphocholine (DPPC) et la monostéaroylphosphatidylcholine (MSPC) ; ou
(E) le liposome comprend en outre un composé choisi dans le groupe comprenant des composés thérapeutiques et des composés d'imagerie, éventuellement
- (i) dans lequel le liposome comprend une bicouche et définit un espace intérieur et le composé se situe dans l'espace intérieur du liposome, à l'intérieur de la bicouche du liposome, ou à la fois dans l'espace intérieur et à l'intérieur de la bicouche ; ou
- (ii) dans lequel le composé thérapeutique est un composé anticancéreux dans lequel, éventuellement, le composé anticancéreux est choisi dans le groupe comprenant des agents alkylants, des antimétabolites, des alcaloïdes végétaux qui sont des poisons fusoriaux, des antibiotiques cytotoxiques anti-tumoraux, des inhibiteurs de topoisomérases, des anticorps monoclonaux ou des fragments de ceux-ci, des photosensibilisateurs, des inhibiteurs de kinases, des enzymes anti-tumorales et des inhibiteurs d'enzymes, des inducteurs de l'apoptose, des modificateurs de la réponse biologique, des anti-hormones, des rétinoïdes et des composés contenant du platine ou dans lequel le composé anti-cancéreux est choisi dans le groupe comprenant le docétaxel, la doxorubicine, et la carboplatine ; ou
- (iii) dans lequel le liposome comprend un agent d'imagerie ; ou
- (iv) dans lequel le liposome comprend la 1,2-distéaroyl-sn-glycéro-3-phosphoéthanolamine-N-[méthoxy(polyéthylène glycol)-2000] (DSPE-PEG2000)-LARLLT, dans lequel l'agent thérapeutique est la carboplatine.

7. Composé destiné à être utilisé en imagerie associé à un peptide selon la revendication 1 ou 2.

8. Composé selon la revendication 7, dans lequel :
(A) le peptide est lié à un lipide et le lipide lié au peptide se trouve sous la forme d'un liposome ; ou
(B) le lipide lié à un peptide comprend une séquence de liaison entre le lipide et le peptide, éventuellement ;
- (i) dans lequel le lipide est la 1,2-distéaroyl-sn-glycéro-3-phosphoéthanolamine (DSPE) dans lequel, éventuellement, la séquence de liaison comprend du polyéthylène glycol (PEG) ; ou
- (ii) dans lequel le lipide lié au peptide comprend la 1,2-distéaroyl-sn-glycéro-3-phosphoéthanolamine-N-[méthoxy(polyéthylène glycol)-2000] (DSPE-PEG2000)-LARLLT ; ou
(C) le liposome comprend en outre la 1,2-dipalmitoyl-sn-glycéro-3-phosphocholine (DPPC) et la monostéaroylphosphatidylcholine (MSPC), et dans lequel le liposome présente une température de transition de phase gel à liquide allant d'environ 39,0 °C à environ 45 °C ; ou
(D) le liposome comprend en outre un lipide substitué par le PEG ; ou
(E) le liposome comprend une bicouche et définit un espace intérieur et le composé se situe dans l'espace intérieur du liposome, à l'intérieur de la bicouche du liposome, ou à la fois dans l'espace intérieur et à l'intérieur de la bicouche.

9. Liposome destiné à être utilisé dans le traitement du cancer chez un sujet qui en a besoin, dans lequel le liposome comprend un lipide associé à un peptide selon la revendication 1 ou 2, et le liposome comprend un composé anti-cancéreux.

10. Liposome destiné à être utilisé selon la revendication 9, dans lequel le peptide est lié au lipide dans lequel, éventuellement, le lipide lié au peptide comprend une séquence de liaison entre le lipide et le peptide, ou dans lequel le lipide est la 1,2-distéaroyl-sn-glycéro-3-phosphoéthanolamine (DSPE), ou dans lequel la séquence de liaison comprend du polyéthylène glycol (PEG), ou dans lequel le lipide lié au peptide comprend la 1,2-distéaroyl-sn-glycéro-3-phosphoéthanolamine-N-[méthoxy(polyéthylène glycol)-2000] (DSPE-PEG2000)-LARLLT.

11. Liposome destiné à être utilisé selon la revendication 9, dans lequel le liposome comprend en outre la 1,2-dipalmitoyl-sn-glycéro-3-phosphocholine (DPPC) et la monostéaroylphosphatidylcholine (MSPC), et dans lequel le liposome présente une température de transition de phase gel à liquide allant d'environ 39,0 °C à environ 45 °C, ou dans lequel le liposome comprend un lipide substitué par le PEG, ou dans lequel le liposome comprend une bicouche et définit un espace intérieur et le composé anti-cancéreux se situe dans l'espace intérieur du liposome, à l'intérieur de la bicouche du liposome, ou à la fois dans l'espace intérieur et à l'intérieur de la bicouche, ou dans lequel le composé anti-cancéreux est choisi dans le groupe comprenant des agents alkylants, des antimétabolites, des alcaloïdes végétaux qui sont des poisons fusoriaux, des antibiotiques cytotoxiques anti-tumoraux, des inhibiteurs de topoisomérases, des anticorps monoclonaux ou des fragments de ceux-ci, des photosensibilisateurs, des inhibiteurs de kinases, des enzymes anti-tumorales et des inhibiteurs d'enzymes, des inducteurs de l'apoptose, des modificateurs de la réponse biologique, des anti-hormones, des rétinoïdes et des composés contenant du platine, ou dans lequel le composé anti-cancéreux est choisi dans le groupe comprenant le docétaxel, la doxorubicine, et la carboplatine.

12. Liposome destiné à être utilisé selon la revendication 9, dans lequel le traitement comprend le chauffage d'une partie du sujet dans lequel, éventuellement, le chauffage comprend l'application d'une énergie électromagnétique, de rayonnements infrarouges, ou d'ultrasons.

13. Peptide selon la revendication 1 ou 2, molécule selon la revendication 3 ou 4, ou liposome selon la revendication 5 ou 6 destinés à être utilisés en thérapie.

14. Peptide, molécule ou liposome selon la revendication 13, dans laquelle ladite thérapie est un traitement du cancer.
